(19) **Europäisches Patentamt**
**European Patent Office**
**Office européen des brevets**

(11) **EP 3 206 013 A1**

(12) **EUROPEAN PATENT APPLICATION**

(43) Date of publication:
**16.08.2017 Bulletin 2017/33**

(21) Application number: **17153495.1**

(22) Date of filing: **27.01.2017**

(51) Int Cl.:
**G01N 21/11** *(2006.01)*          **G01N 21/64** *(2006.01)*
**G01N 21/76** *(2006.01)*          **G01N 35/10** *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **31.01.2016   JP 2016016782
23.01.2017   JP 2017009175**

(71) Applicant: **ARKRAY, Inc.
Minami-ku
Kyoto-shi,
Kyoto 601-8045 (JP)**

(72) Inventors:
• **Matsumoto, Daisuke
Kyoto, 602-0008 (JP)**
• **Tanaka, Takashige
Kyoto, 602-0008 (JP)**
• **Odagaki, Toru
Kyoto, 602-0008 (JP)**

(74) Representative: **Hoffmann Eitle
Patent- und Rechtsanwälte PartmbB
Arabellastraße 30
81925 München (DE)**

(54) **ANALYSIS TOOL AND ANALYSIS DEVICE**

(57)      An analysis tool for use mounted to an analysis device that automatically analyzes a specified component contained in a sample. The analysis tool includes a light measurement well to hold a measurement solution as a measurement subject, and to measure the measurement solution. The light measurement well includes an opening to dispense the measurement solution through, a measurement solution holder to hold the measurement solution dispensed through the opening, and an emission section that emits measurement light caused to be emitted from the measurement solution held in the measurement solution holder in a light receiving direction of the analysis device. The measurement light is fluorescent light or chemiluminescent light. The measurement solution holder has a flattened profile that is flattened in the light receiving direction.

EP 3 206 013 A1

**Description**

BACKGROUND

Technical Field

Technology disclosed herein relates to an analysis tool and analysis device capable of high precision analysis of a specified component in a sample by detecting fluorescent light or chemiluminescent light with high sensitivity.

Related Art

Hitherto, fluorescence analysis devices, after regulating to collect excitation light into a beam using a condenser, then use an optical filter to illuminate selected wavelengths into a sample container. Fluorescent light emitted from the sample container is then collected and regulated to form a beam using an optical lens, then wavelengths of light are selected using an optical filter for detection with a light detector. However, only some of the fluorescent light, which is emitted in all directions, can be captured in this method, so the light collection cannot be said to be efficient. Similar issues arise in analysis devices that measure chemiluminescent light or bioluminescence.

In response, for example, Japanese Patent Application Laid-Open (JP-A) Nos. H10-019779 and 2000-241708 describe technology for collecting light, such as fluorescent light, scattered in many directions from a sample container. JP-ANo. H10-019779 describes a fluorescence analysis device including a condenser mirror that uses reflection to collect fluorescent light scattered from a sample container. JP-A No. 2000-241708 describes a light emission analysis device including a detector for detecting light emitted from a light collecting element with a light reflecting surface at the periphery of a sample container. These devices are capable of capturing light emitted in many directions.

However, in the related technology, there is room for improvement in the following points.

In the fluorescence analysis device of JP-A No. H10-019779, the actual light collecting efficiency is not especially high when attenuation of the fluorescent light on reflection by the condenser mirror, and light collecting efficiency using a lens system, are considered. Moreover, there is an increase in size due to the complexity of the structure of the optical system. In the light emission analysis device of JP-A No. 2000-241708, there is also a large amount of attenuation, arising due to repeated reflection. The light collecting efficiency is therefore not especially high.

SUMMARY

In consideration of the above circumstances, the technology disclosed herein provides an analysis tool and analysis device capable of precise analysis of a specified component in a sample by improving light collecting efficiency.

An analysis tool provided by a first aspect of technology disclosed herein is an analysis tool for use mounted to an analysis device that automatically analyzes a specified component contained in a sample. The analysis tool includes a light measurement well to hold a measurement solution as a measurement subject, and to measure the measurement solution. The light measurement well includes an opening to dispense the measurement solution through, a measurement solution holder to hold the measurement solution dispensed through the opening, and an emission section that emits measurement light caused to be emitted from the measurement solution held in the measurement solution holder in a light receiving direction of the analysis device. The measurement light is fluorescent light or chemiluminescent light. The measurement solution holder has a flattened profile that is flattened in the light receiving direction.

Preferably, the light measurement well is formed flattened overall in a shape matching that of the measurement solution holder and including a flattened face, and the emission section is provided at the flattened face.

Preferably, a height of the measurement solution in a crosswise direction of a cross-section of the flattened profile determines a cell length of the light measurement well, and the cell length is 3 mm or less.

Preferably, the analysis tool further includes a reaction tube to generate the measurement solution.

Preferably, in cases in which the measurement light is fluorescent light, excitation light for causing the measurement light to be emitted is illuminated onto the measurement solution from a portion other than the emission section of the light measurement well.

Preferably, an illumination direction of the excitation light is aligned with the light receiving direction of the measurement light.

An analysis device provided by a second aspect of technology disclosed herein is an analysis device employing the analysis tool of the first aspect of technology disclosed herein. The analysis device includes a detector that detects the measurement light.

Preferably, the analysis device further includes a measurement light wavelength selection filter to limit wavelengths of the measurement light.

Preferably, the measurement light wavelength selection filter has predetermined wavelength absorption characteristics.

Preferably, the measurement light wavelength selection filter is a colored glass filter.

Preferably, the analysis device further includes a light collecting member to collect the measurement light.

Preferably, the light collecting member is a light guide.

Preferably, in cases in which the measurement light is the fluorescent light, the analysis device further includes a light source that illuminates excitation light to cause the measurement solution to emit fluorescent light, and an excitation light wavelength selection filter to limit wavelengths of the excitation light.

Preferably, the excitation light wavelength selection filter has predetermined wavelength absorption characteristics.

Preferably, the excitation light wavelength selection filter is a colored glass filter.

Preferably, the analysis tool is configured such that a cell length of the light measurement well is determined by a height of the measurement solution in a crosswise direction of a cross-section of the flattened profile, and can be changed by an amount of the measurement solution, and the analysis device further includes: a nozzle; and a controller that controls operation of the nozzle and the detector, the controller being configured so as to, in cases in which an output value of the detector has become saturated, reduce the amount of the measurement solution by drawing the measurement solution into the nozzle, and to then cause the detector to re-execute measurement.

Preferably the analysis device is configured such that the amount by which to reduce the amount of the measurement solution is determined based on time taken from detecting the measurement light with the detector until the output value of the detector reaches a saturated state.

Preferably the analysis device is configured such that the amount by which to reduce the amount of the measurement solution is determined based on time taken from detecting the measurement light with the detector until the output value of the detector reaches a saturated state, and a rate of rise of the output value of the detector during a predetermined period that has been predetermined as an initial reaction period of the measurement solution.

An analysis tool according to a third aspect of technology disclosed herein is an analysis tool for use mounted to an analysis device that automatically analyzes a specified component contained in a sample. The analysis tool includes: a first base plate including a solution transfer opening; a second base plate stacked on the first base plate; a reaction flow path that is formed between the first base plate and the second base plate, and that is linked to the solution transfer opening; and a light measurement well that is provided at the reaction flow path, that generates a measurement solution as a measurement subject, and that emits measurement light caused to be emitted from the measurement solution in a light receiving direction of the analysis device. The solution transfer opening is employed to move the measurement solution back and forth in the reaction flow path by drawing and discharging air. The measurement light is fluorescent light, chemiluminescent light, or transmitted light. The light measurement well has a flattened profile that is flattened in the light receiving direction.

Preferably, an antibody or an antigen for the specified component is immobilized in the light measurement well.

Preferably, immobilized magnetic particles, on which an antibody or an antigen for the specified component has been immobilized, are disposed in the light measurement well.

An analysis device according to a fourth aspect of technology disclosed herein is an analysis device employing the analysis tool of the third aspect of technology disclosed herein. The analysis device includes a detector to detect the measurement light.

An analysis device according to a fifth aspect of technology disclosed herein is an analysis device employing the analysis tool of the third aspect of technology disclosed herein, wherein immobilized magnetic particles, on which an antibody or an antigen for the specified component has been immobilized, are disposed in the light measurement well, and the analysis device includes a detector to detect the measurement light, and a magnet to generate magnetic force to keep the immobilized magnetic particles in the light measurement well, the magnet being placed close to the light measurement well.

In the technology disclosed herein, light collecting efficiency is improved, enabling high precision analysis of a specified component in a sample.

Other characteristics and advantages of the technology disclosed herein are made clear in the following explanation of embodiments of the invention, with reference to the attached drawings.

BRIEF DESCRIPTION OF THE DRAWINGS

Embodiments of the present disclosure will be described in detail based on the following figures, wherein:

Fig. 1 is a schematic configuration diagram illustrating an example of an analysis system including an analysis tool and an analysis device according to a first embodiment of technology disclosed herein;
Fig. 2A is a perspective view illustrating an example of an analysis tool configuring the analysis system illustrated in Fig. 1;

Fig. 2B is an exploded perspective view illustrating of the analysis tool illustrated in Fig. 2A when disassembled;

Fig. 3A is a vertical cross-section taken along a crosswise direction of a reaction tube configuring the analysis tool illustrated in Fig. 2A;

Fig. 3B is a vertical cross-section taken along a lengthwise direction of a reaction tube configuring the analysis tool illustrated in Fig. 2A;

Fig. 4A is a perspective view of a light measurement well configuring the analysis tool illustrated in Fig. 2A;

Fig. 4B is a cross-section taken along line IVB-IVB in Fig. 4A;

Fig. 5A is a cross-section illustrating a viewing angle at an uppermost portion of a measurement solution held in the light measurement well illustrated in Fig. 4A;

Fig. 5B is a cross-section illustrating a viewing angle at an uppermost portion of a measurement solution when the same amount of measurement solution as in Fig. 5A is held in a light measurement well with a longer cell length;

Fig. 6 is a graph illustrating measurement results for transmissivity characteristics of 4-MUP solution (0.6 mM), this being a fluorogenic substrate solution, employing a 10 mm cell;

Fig. 7 is a cross-section illustrating an example of a detection section configuring the analysis system illustrated in Fig. 1;

Fig. 8 is a cross-section to explain an example of a solution transfer operation of an analysis device configuring the analysis system illustrated in Fig. 1;

Fig. 9A is a perspective view illustrating an analysis tool according to a second embodiment of technology disclosed herein;

Fig. 9B is a cross-section taken along line IXB-IXB in Fig. 9A;

Fig. 10A is a cross-section illustrating a back and forth movement state in the analysis tool illustrated in Fig. 9A;

Fig. 10B is a cross-section illustrating a state when measuring a measurement solution using the analysis tool illustrated in Fig. 9A;

Fig. 11 is a cross-section illustrating an analysis tool according to a third embodiment of technology disclosed herein;

Fig. 12A is a cross-section illustrating an analysis tool according to a fourth embodiment of technology disclosed herein;

Fig. 12B is a cross-section illustrating a state when measuring a measurement solution using the analysis tool illustrated in Fig. 12A;

Fig. 13 is a cross-section illustrating an analysis tool according to a fifth embodiment of technology disclosed herein;

Fig. 14 is a graph illustrating a relationship between cell length and fluorescent light harvesting rate in an Example 1 of technology disclosed herein;

Fig. 15 is a graph illustrating results of confirming the presence or absence of the prozone effect due to cell length in an Example 2 of technology disclosed herein;

Fig. 16 is a graph illustrating a relationship between cell length and a dynamic range high limit in an Example 3 of technology disclosed herein;

Fig. 17 is a block diagram illustrating an example of a hardware configuration in an electrical system of an analysis device according to the first embodiment of technology disclosed herein;

Fig. 18 is a graph illustrating an example of a first behavior pattern of output values of a light receiving element included in an analysis device according to the first embodiment of technology disclosed herein;

Fig. 19 is a graph illustrating an example of a second behavior pattern of output values of a light receiving element included in an analysis device according to the first embodiment of technology disclosed herein;

Fig. 20 is a flowchart illustrating an example of a flow of measurement processing according to the first embodiment of technology disclosed herein;

Fig. 21 is a flowchart illustrating a modified example of a flow of measurement processing according to the first embodiment of technology disclosed herein; and

Fig. 22 is a schematic view illustrating an example of a configuration in which a measurement program according to the first embodiment of technology disclosed herein is installed in an analysis device from a storage medium stored with the measurement program.

DETAILED DESCRIPTION

Specific explanation follows regarding a preferable embodiment of technology disclosed herein, with reference to the drawings. Note that in the following explanation, directions such as the vertical direction correspond to the notation in the drawings. Moreover, "to" in the context of numerical ranges indicates a range including the values before and after "to" as minimum values and maximum values, respectively.

First Embodiment

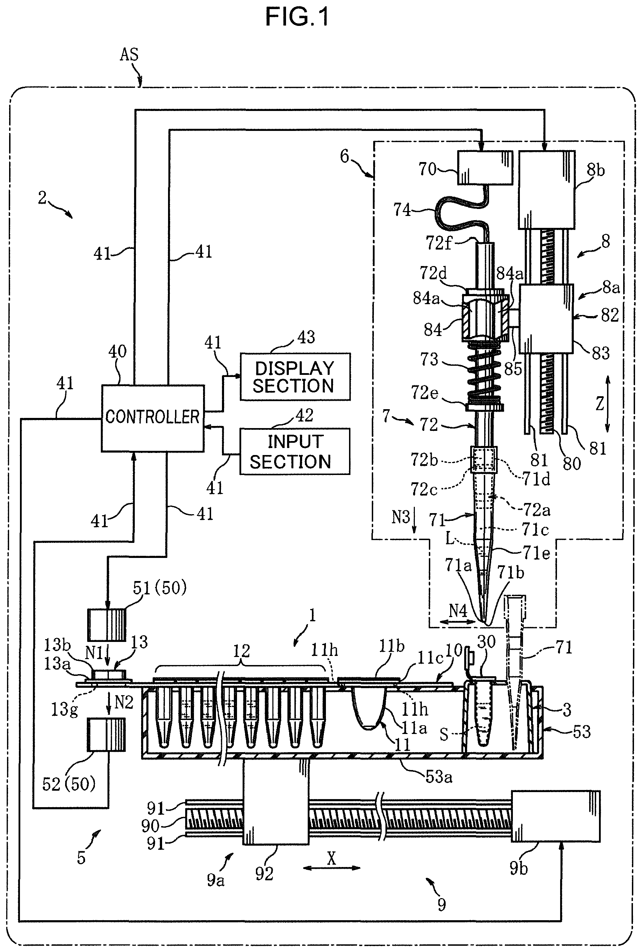

An analysis system AS applied with an analysis tool 1 and an analysis device 2 of the technology disclosed herein is installed in a hospital or veterinary clinic, and is employed to analyze a specified component included in a biological sample S using fluorescence analysis or chemiluminescence analysis. As illustrated in Fig. 1, the analysis system AS includes the analysis tool 1 and the analysis device 2. The biological sample S corresponds to an example of a sample of technology disclosed herein. A liquid, such as bodily fluid, or a solid, such as feces, is employed as the biological sample S. When using a liquid sample, pre-processing is performed to dilute the sample with a diluent as needed. When analyzing a solid sample, pre-processing is performed to dissolve the sample in a solvent, or to suspend the sample in a suspension liquid, if necessary. Specific examples of the biological sample S are human or animal blood, urine, saliva, serum, blood plasma, and feces.

Analysis Tool

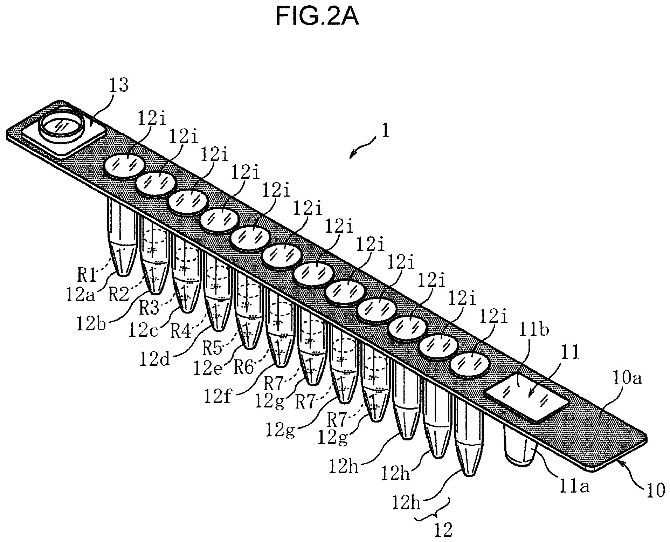

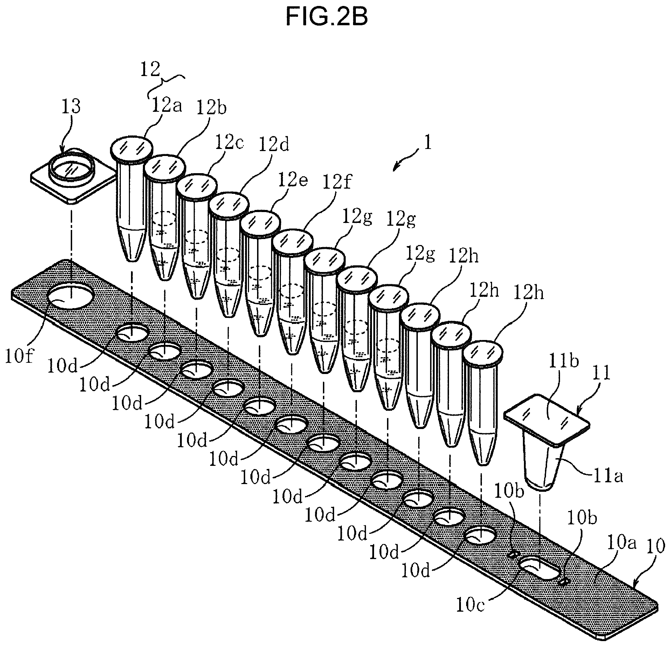

As illustrated in Fig. 2A and Fig. 2B, the analysis tool 1 includes, for example, a base plate 10, a reaction tube 11, plural tubes 12, and a light measurement well 13. The analysis tool 1 is, for example, used to inspect a specified component in the biological sample S using an immunoassay method. An example of the immunoassay method used is an enzyme immunoassay. An example of the enzyme immunoassay used is an ELISA technique. An example of the ELISA technique used is the sandwich method. Examples of the specified component for analysis are rheumatoid factor (RF), carcinoembryonic antigen (CEA), alpha-fetoprotein (AFP), or HIV antibody. These are contained in serum, this being an example of the biological sample S.

As illustrated in Fig. 2B, the reaction tube 11, the plural tubes 12, and the light measurement well 13 are integrally mounted to the base plate 10. As illustrated in Fig. 2A and Fig. 2B, the surface of the base plate 10 is colored black or has a black sticker adhered thereto in order to prevent light from leaking out from an optical system 50, described later. The base plate 10 is formed from a synthetic resin. Specific examples of the synthetic resin used include polystyrene (PS), poly (methylmethacrylate) (PMMA), polyethylene terephthalate (PET), polycarbonate (PC), polyethylene (PE), and polypropylene (PP). The base plate 10 includes a mounting hole 10c for mounting the reaction tube 11, mounting holes 10d for mounting the plural tubes 12, and a mounting hole 10f for mounting the light measurement well 13.

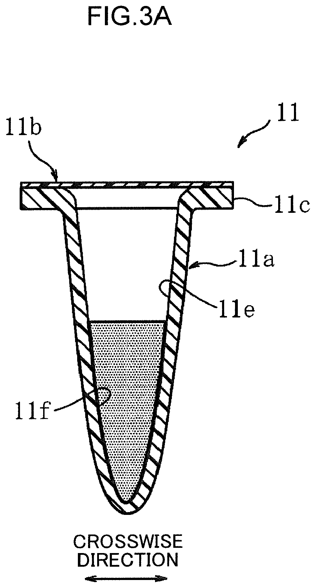

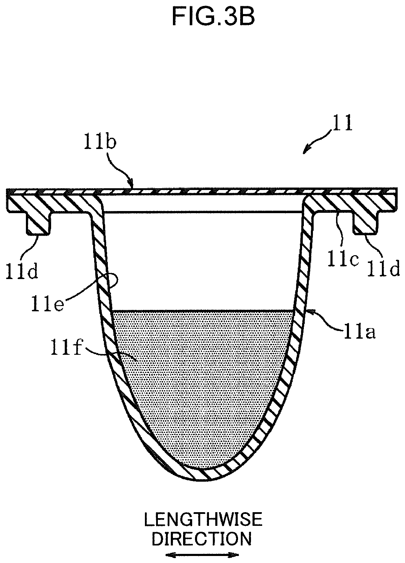

The reaction tube 11 is a container to perform analytical reactions in, in order to analyze a specified component in the biological sample S, by sequentially swapping in plural types of liquid (R1 to R6). As an example, as illustrated in Fig. 3A and Fig. 3B, the reaction tube 11 includes a main body 11a and a seal 11b. The main body 11 a is made from a synthetic resin. A specific example of the synthetic resin used is polystyrene (PS). Other than polystyrene, examples include transparent or translucent resins such as poly (methyl methacrylate) (PMMA), cyclo-olefin polymer (COP), polyethylene terephthalate (PET), polycarbonate (PC), low density polyethylene (LDPE), polylactic acid (PLA), polydimethylsiloxane (PDMS), and polypropylene (PP).

The seal 11b is adhered to an upper face of a flange 11 c of the main body 11 a. The seal 11b is made from, for example, aluminum foil, a multilayered film including aluminum foil, or a synthetic resin film. The seal 11b is formed so as to be pierceable by a pipette tip leading end 71 a, described later. The main body 11a and the seal 11b are, for example, adhered together by thermal welding. In the example illustrated in Fig. 3b, the reaction tube 11 includes fitting projections 11d at a lower face of the flange 11c. As illustrated in Fig. 1, Fig. 2A, and Fig. 2B as an example, the reaction tube 11 is mounted to the base plate 10 by inserting the main body 11a into the mounting hole 10c, and fitting the fitting projections 11d into fitting holes 10b.

As illustrated as an example in Figs. 3A and 3B, an inner face 11e of the main body 11a is configured with an immobilized antibody for the specified component. A monoclonal antibody or a polyclonal antibody is employed as the immobilized antibody 11f. The immobilized antibody 11f is, for example, goat, mouse, horse, bovine, chicken, dog, human, porcine, rabbit, rat, Syrian hamster, or Xenopus in origin. Antibody immobilization on the inner face 11 e of the main body 11 a is performed using a normal method. The reaction tube 11 is mounted to the base plate 10 after performing the antibody immobilization. Configuration may also be made in which rather than immobilizing the antibody on the inner face 11e of the main body 11a, an antibody-sensitized magnetic particle solution is prepared as a separate reagent.

It is difficult to immobilize antibodies by physical adsorption on some of the synthetic resins which may be selected as the material of the main body 11a. In such cases, after performing VUV processing, plasma processing, chemical processing, or the like, carboxyl groups or amino groups are introduced to the inner face 11e of the main body 11a, and the antibody is immobilized by covalent bonding to these functional groups. Alternatively, immobilization may be performed on a coating such as a self-assembled monolayer (SAM).

In the examples illustrated in Fig. 1, Fig. 2A, and Fig. 2B, the plural tubes 12 each include a biological sample dilution tube 12a, a biological sample dilution tube 12b, a primary antibody solution tube 12c, a secondary antibody solution tube (enzyme marked antibody solution tube) 12d, an enzyme substrate solution tube 12e, a reaction-stop solution tube 12f, buffer wash solution tubes 12g, and waste solution tubes 12h. The plural tubes 12 are formed from a synthetic resin. Specific examples of the synthetic resin include polystyrene (PS), poly (methyl methacrylate) (PMMA), polyethylene terephthalate (PET), polycarbonate (PC), polyethylene (PE), and polypropylene (PP). Seals 12i are adhered to upper faces of the tubes. The seals 12i are made from, for example, aluminum foil, multilayered film including aluminum foil, or synthetic resin film, and are formed so as to be pierceable by the pipette tip leading end 71 a, described later. As illustrated in Fig. 2B as an example, the plural tubes 12 are mounted to the base plate 10 by being fitted into the mounting holes 10d.

A predetermined amount of the biological sample S is dispensed into the biological sample dilution tube 12a, and the biological sample dilution tube 12a is used to prepare a mixture solution R1 diluted to an appropriate concentration. The biological sample dilution tube 12b is a tube filled with a biological sample dilution solution R2 for diluting the biological sample S. The biological sample dilution solution R2 is used to dilute the biological sample S dispensed into the biological sample dilution tube 12a to a predetermined concentration. A phosphoric acid buffer solution, for example, is employed as the biological sample dilution solution R2.

The primary antibody solution tube 12c is a tube that holds a primary antibody solution R3. Similarly to the immobilized antibody 11f, the primary antibody is an antibody for the specified component, and a monoclonal antibody or a polyclonal antibody is employed. Similarly to the immobilized antibody 11f, the primary antibody is, for example, obtained from an animal mentioned above. The primary antibody is, for example, dissolved in a phosphoric acid buffer solution.

The secondary antibody solution tube 12d is a tube that holds a secondary antibody (enzyme marked antibody) solution R4. The enzyme marked antibody is, for example, dissolved in a phosphoric acid buffer solution. The secondary antibody is an antibody for the primary antibody, and a monoclonal antibody or a polyclonal antibody may be employed. Similarly to the immobilized antibody 11f, the secondary antibody may, for example, be obtained from an animal described above. The secondary antibody may be marked using horseradish peroxidase (HRP). The secondary antibody may also be marked using alkaline phosphatase (AP), for example.

The enzyme substrate solution tube 12e is a tube that holds an enzyme substrate solution R5, as a reagent for detecting the specified component. Examples of the enzyme substrate include a fluorogenic substrate or a chemiluminescent substrate. Hydrogen peroxide ($H_2O_2$) is added in addition to the above when the marker enzyme is HRP. The enzyme substrate solution R5 is adjusted to a predetermined pH depending on its type. Note that $H_2O_2$ may be prepared as a separate reagent.

The fluorogenic substrate is employed in fluorescent light detection for the specified component. In fluorescent light detection, the presence or absence of, and the amount of, the specified component is detected by detecting fluorescent light emitted when a fluorophore, which is generated when the fluorogenic substrate is cleaved by the marker enzyme, is illuminated with excitation light. When the marker enzyme is HRP, specific examples of the fluorogenic substrate include 4-hydroxy-3-methoxy phenylacetic acid, reduced phenoxazine, reduced benzothiazine, and reduced dihydroxanthene. When the marker enzyme is AP, specific examples of the fluorogenic substrate include 4-methylumbelliferyl phosphate (4-MUP), 2-(5'-chloro-2'-phosphoryloxyphenyl)-6-chloro-4-(3H)-quinazolinone (CPPCQ), 3,6-fluorescein diphosphate (3,6-FDP), Fast Blue BB(FastBlue-BB), Fast Red TR, and Fast Red Violet LB diazonium salt.

On the other hand, the chemiluminescent substrate is employed in chemiluminescence detection for the specified component. In chemiluminescence detection, the presence or absence of, and the amount of, the specified component is detected by detecting chemiluminescent light emitted by the chemiluminescent substrate when the chemiluminescent substrate is cleaved by the marker enzyme. When the marker enzyme is HRP, specific examples of the chemiluminescent substrate include, for example, chemiluminescent substrates having a luminol base. When the marker enzyme is AP, specific examples of the chemiluminescent substrate include 3-(2'-spiroadamantane)-4-methoxy-4-(3'-phosphoryloxy)phenyl-1,2-dioxetane disodium salt (AMPPD), 2-chloro-5-{4-methoxyspiro [1,2-dioxetane-3,2'-(5'-chloro)tricyclo [3.3.1.1$^{3,7}$] decane ]-4-yl} phenylphosphate disodium salt (CDP-Star (registered trademark)), 3-{4-methoxyspiro [1,2-dioxetane-3,2'-(5'-chloro)tricyclo [3.3.1.1$^{3,7}$]decane ]-4-yl} phenylphosphate disodium salt (CSPD (registered trademark)), [10-methyl-9(10H)-acridinylidene] phenoxymethyl phosphoric acid disodium (Lumigen (registered trademark), APS-5), and 9-(4-chlorophenylthiophosphoryloxymethylidene )-10-methylacridine disodium salt.

The reaction stop solution tube 12f is a tube for holding a reaction stop solution R6. The reaction stop solution R6 is used to stop the secondary antibody marker enzyme and the enzyme substrate from reacting with each other. An aqueous solution of sulfuric acid or an aqueous solution of sodium hydroxide may be employed as the reaction stop solution R6.

The buffer wash solution tubes 12g are tubes for holding a buffer wash solution R7. The buffer wash solution R7 is for washing a pipette tip 71, and is prepared with a solution to wash the reaction tube 11. A phosphoric acid buffer solution or a tris buffer solution, for example, may be employed as the buffer wash solution R7. The surfactant TWEEN 20 (registered trade mark) is added to the buffer solution. Plural of the buffer wash solution tubes 12g may be provided, according to the amount to be used.

The waste solution tubes 12h are tubes for placing the reagent solutions (R1 to R6) used in the reaction tube 11, or the buffer wash solution R7, as waste solution R8, described later. Plural of the waste solution tubes 12h may be provided, according to the amount of waste solution.

Note that hereafter, the mixture solution R1, the biological sample dilution solution R2, the primary antibody solution R3, the secondary antibody solution R4, the enzyme substrate solution R5, the reaction stop solution R6, and the buffer wash solution R7 are referred to collectively as liquids L.

The light measurement well 13 is a vessel into which a predetermined amount of a measurement solution L1, arising at the end of reactions when the liquid L in the reaction tube 11 is swapped in sequence, is dispensed. The light measurement well 13 is used to measure measurement light such as fluorescent light or chemiluminescent light emitted from the measurement solution L1. The light measurement well 13 is formed from a transparent synthetic resin. Examples of materials employed as the synthetic resin include polystyrene (PS), poly (methylmethacrylate) (PMMA), polyethylene terephthalate (PET), polycarbonate (PC), polyethylene (PE), and polypropylene (PP).

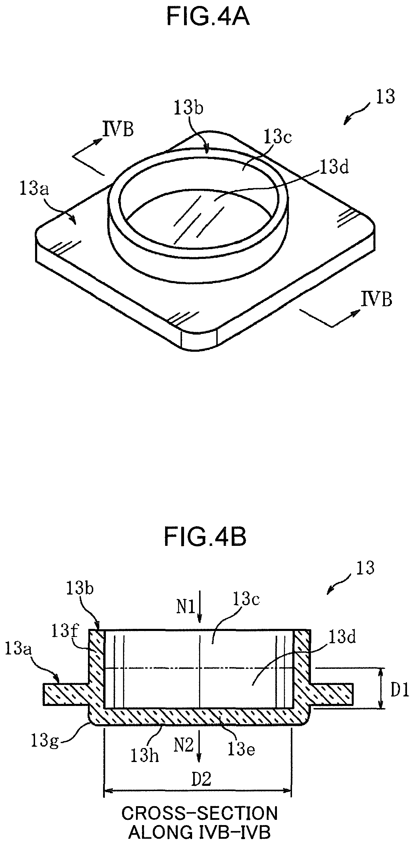

As illustrated as an example in Fig. 4A and Fig. 4B, the light measurement well 13 includes a flange 13a and a well body 13b. The well body 13b includes an upper opening 13c, a measurement solution holder 13d, a bottom wall 13e, and a side wall 13f. The bottom wall 13e is included in a downward protrusion 13g.

The downward protrusion 13g is fitted into the mounting hole 10f provided at the base plate 10As illustrated as an example in Fig. 2B, thereby mounting the light measurement well 13 on the base plate 10. As illustrated as an example in Fig. 4A and Fig. 4B, the upper opening 13c is a dispensing opening for dispensing the measurement solution L1 into. The upper opening 13c corresponds to an example of an opening of technology disclosed herein. The measurement solution holder 13d is a portion for holding the measurement solution L1. The measurement solution holder 13d is formed so as to be surrounded by the bottom wall 13e and the side wall 13f. The upper opening 13c and the measurement solution holder 13d are circular shaped in plan view. Note that the base plate 10, the reaction tube 11, the plural tubes 12, the light measurement well 13, and a micro tube 30 holding the biological sample S, may integrated together in any appropriate combination.

As illustrated as an example in Fig. 4B, when using the light measurement well 13 with fluorescent light detection, excitation light is illuminated along an illumination direction N1 directly through the wall-less upper opening 13c. Fluorescent light emitted along a light receiving direction N2 is detected through a lower face 13h of the bottom wall 13e. Accordingly, fluorescent light detection using the light measurement well 13 is performed by TOP-BOTTOM light measurement. In such a configuration, the excitation light illumination direction N1 and the light receiving direction N2 of the fluorescent light are aligned with each other. Fluorescent light corresponds to an example of measurement light of technology disclosed herein. When the light measurement well 13 is used to detect chemiluminescent light, chemiluminescent light emitted through the lower face 13h of the bottom wall 13e is detected. Chemiluminescent light corresponds to an example of measurement light of technology disclosed herein.

As illustrated as an example in Fig. 4B, the shape of the measurement solution holder 13d is a profile flattened in the light receiving direction N2. In the light measurement well 13, the shape of the measurement solution holder 13d is, for example, determined by the height (depth) of the measurement solution L1 dispensed into the well body 13b, and the internal diameter of the well body 13b. The height (depth) of the measurement solution holder 13d is a cell length D1 of the light measurement well 13. Specifically, the height (depth) of the measurement solution L1 dispensed into the measurement solution holder 13d in the crosswise direction of the cross-section of the flattened profile measurement solution holder 13d defines (determines) the cell length D1 of the light measurement well 13. Moreover, the internal diameter of the well body 13b is given by a diameter D2 of the measurement solution holder 13d. Note that the shapes of the well body 13b and the light measurement well 13 may be configured overall with flattened profiles matching the shape of the measurement solution holder 13d. In such cases, the lower face 13h of the bottom wall 13e is a flattened face. This flattened face is not limited to being a flat horizontal face, and may be a gently undulating face, or a curved face. Note that the lower face 13h corresponds to an example of an emission section of technology disclosed herein.

The cell length D1 of the light measurement well 13 is, for example, 3.0 mm or less, 1.5 mm to 3.0 mm, 1.9 mm to 2.5 mm, 2.5 mm, 2.0 mm, 1.5 mm to 2.0 mm, 1.9 mm to 2.0 mm, 2.0 mm to 2.5 mm, 2.0 mm to 3.0 mm, or 2.0 mm or less. The diameter D2 of the light measurement well 13 is, for example, 8.0 mm or less, 8.0 mm to 11.3 mm, 8.8 mm to 10.0 mm, 9.8 mm, 10.0 mm, 9.8 mm to 10.0 mm, 8.8 mm to 9.8 mm, 9.8 mm to 10.0 mm, 8.8 mm to 9.8 mm, or 3.0 mm to 5.0 mm.

The light measurement well 13 of the analysis tool 1 may be configured such that the cell length D1 can be changed by changing the amount of the measurement solution L1 dispensed into the measurement solution holder 13d. The light measurement well 13 thereby configured such that when the output value of a light receiving element 52e is saturated the analysis device 2 re-performs measurements after reducing the amount of the measurement solution L1 and shorting the cell length D1, described later.

Note that when PS is employed as the material for the light measurement well 13, a GPPS grade having low autofluorescence emission strength is employed. Specifically, HF77, HH102, and SGP10 (trade names), manufactured by PS Japan Corporation, are preferable examples of the forming material. Moreover, the bottom wall 13e and the side wall 13f of the light measurement well 13 (locations hit by the excitation light) are made with a thickness of 1.0 mm or less, even if only locally, and preferably made with thickness of 0.5 mm, so as to suppress light scattering and autofluorescence as much as possible.

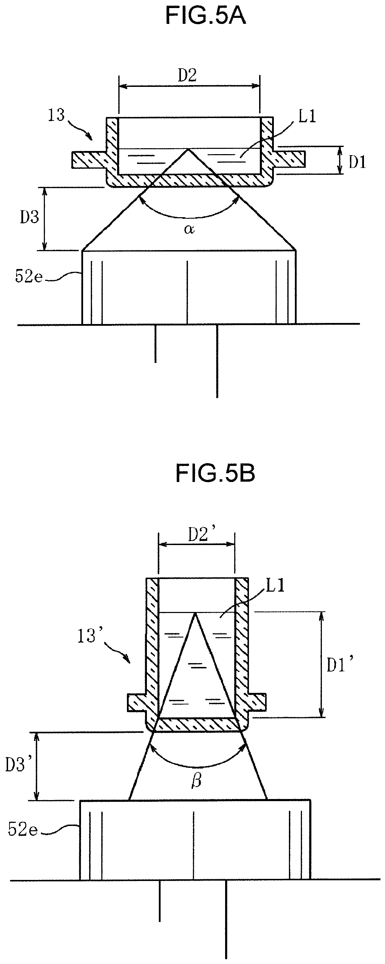

Fig. 5A is a cross-section illustrating an example of a viewing angle $\alpha$ at a fluorescent light emission location at an uppermost portion of the measurement solution L1 held in the light measurement well 13. Fig. 5B is a cross-section illustrating an example of a viewing angle $\beta$ at a fluorescent light emission location at an uppermost portion of the measurement solution L 1 when the same amount of measurement solution L 1 as in the light measurement well 13 in Fig. 5A is held in a light measurement well 13' having a longer cell length. The following relationship holds: cell length D1 < cell length D1'; and diameter D2 > diameter D2'. Moreover, a distance D3 between the light measurement well 13 and the light receiving element 52e is the same as a distance D3' between the light measurement well 13' and a light receiving element 52e'. As is apparent from Fig. 5A and Fig. 5B, the viewing angles have the relationship: viewing angle $\alpha$ > viewing angle $\beta$. Namely, the viewing angle of the fluorescent light or chemiluminescent light is increased by using a shorter cell length. Accordingly, for the same amount of the measurement solution L1, a shorter cell length D1 of the light measurement well 13 is more advantageous due to raising the light collecting efficiency. Moreover, in cases in which the measurement light is fluorescent light, shortening the cell length D1 discourages absorption of the excitation light by the measurement solution L1 that contains a fluorogenic substrate, thereby enabling fluorescent light to be emitted efficiently.

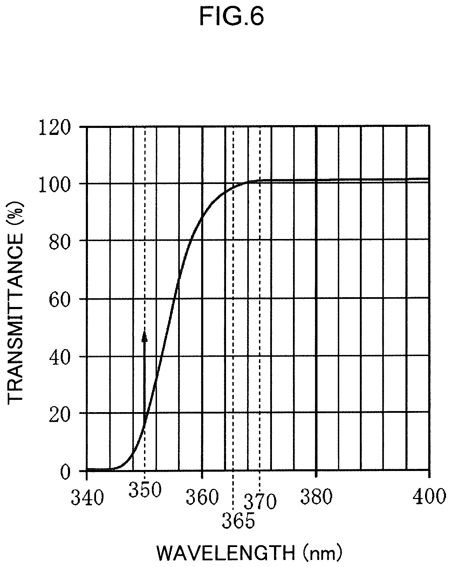

Fig. 6 illustrates an example of results from using a 10 mm cell to measure transmittance characteristics of a 4-MUP solution (0.6 mM), which is an example of a fluorogenic substrate, with a spectrophotometer. The wavelength of excitation light resulting in 4-MU from cleavage of 4-MUP is approximately from 365 nm to 370 nm. As described later, an LED NSHU591B (trade name) manufactured by Nichia Corporation, is, for example, employed as a light emitting element 51a in a detection section 5. The NSHU591B has a central wavelength of 365 nm, a central wavelength error of $\pm 3$ nm, and a spectrum half width of 12 nm. Accordingly, when the central wavelength error is -3 nm, the spectrum half width on the short wavelength side is 350 nm. As is apparent from Fig. 6, transmissivity drops to around 15% at 350 nm. Accordingly, when employing an NSHU591B, excitation light is absorbed by the measurement solution L1 containing 4-MUP. Fluorescent light emission efficiency therefore becomes poorer, and light collecting efficiency could suffer. In such cases, shortening the cell length D1 of the light measurement well 13 enables transmissivity to be raised, as illustrated by the arrow in Fig. 6. This makes the measurement solution L1 containing the fluorogenic substrate less liable to absorb excitation light, thereby enabling efficient fluorescent light emission.

Analysis Device

As illustrated as an example in Fig. 1, the analysis device 2 is a device for analyzing the specified component contained in the biological sample S when the analysis tool 1 has been set at a predetermined location inside the analysis device 2. The analysis device 2 includes a biological sample rack 3, a controller 40, an input section 42, a display section 43, the detection section 5, and a dispensing section 6.

The biological sample rack 3 is a rack on which a micro tube 30 containing the biological sample S is placed, and on which the pipette tip 71 is placed before and after use. The biological sample rack 3 is molded from a synthetic resin. Examples of the synthetic resin used include polystyrene (PS), polycarbonate PC, polyethylene PE, and polypropylene PP. As described later, the biological sample rack 3 is placed on a placement tray 53 together with the analysis tool 1.

As illustrated as an example in Fig. 1, the detection section 5 includes a light source section 51, a light receiving section 52, and an X-axis motor 9b. The dispensing section 6 includes a Z-axis motor 8b and a pump 70.

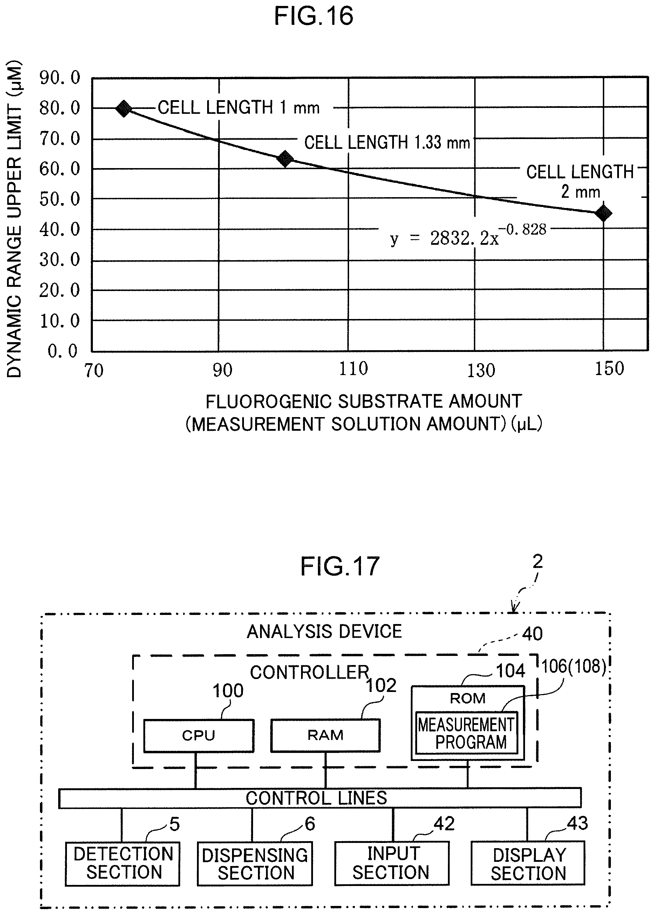

As illustrated as an example in Fig. 1 and Fig. 17, the controller 40 is connected to the dispensing section 6, the detection section 5, the input section 42, and the display section 43 through control lines 41. As illustrated as an example in Fig. 17, the controller 40 includes a central processing unit (CPU) 100; random access memory (RAM) 102; and read-only memory (ROM 104). Note that although explanation is given regarding the ROM 104 as an example, this is merely one example thereof, and non-volatile memory such as Electrically Erasable Programmable Read Only Memory (EEPROM) or flash memory may be employed instead of the ROM 104.

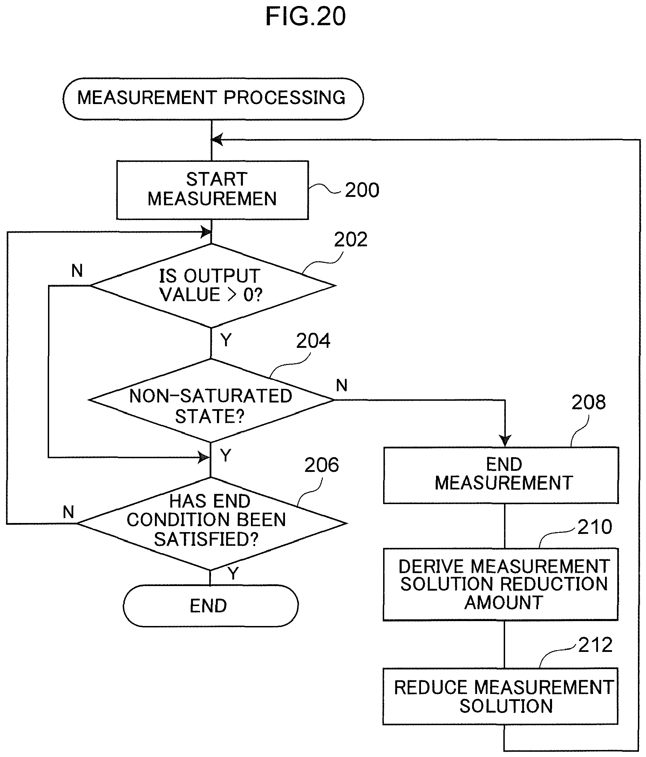

The ROM 104 is stored with various programs, including a measurement program 106 executed in order to implement measurement processing (see Fig. 20), described later, and with various parameters and the like. The CPU 100 reads the various programs from the ROM 104, and performs control processing for the respective sections mentioned above, and performs computation processing on analysis data. The CPU 100 employs the RAM 102 as working memory when performing such processing.

The input section 42 is a section employed for input of required data in analysis, and for selection of selection fields displayed on the display section 43, described later. Specific examples of the input section 42 include a keyboard, a mouse, a touch panel, and a barcode reader. Specific examples of the input data include patient ID numbers, analysis fields, and parameters required in analysis.

The display section 43 displays selection fields required in analysis, and analysis results, for example. Specific examples of the display section include a liquid crystal monitor.

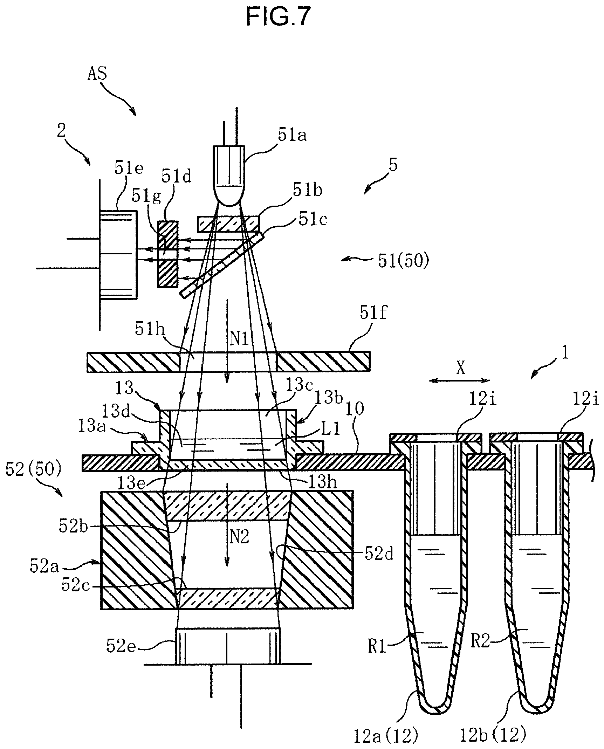

As illustrated as an example in Fig. 1 and Fig. 7, the detection section 5 includes the optical system 50, the placement tray 53, and a horizontal drive section 9. The optical system 50 includes the light source section 51 and the light receiving section 52. In cases in which a fluorogenic substrate is employed as the enzyme substrate, for example, the light source section 51 is used to illuminate excitation light, in the illumination direction labeled N1 onto the light measurement well 13 of the analysis tool 1. The excitation light is illuminated in the illumination direction N1 directly through the wall-less upper opening 13c. The timing of the excitation light illumination is controlled by the controller 40. The light receiving section 52 is used to receive fluorescent light emitted in the light receiving direction, labeled N2, through the bottom wall 13e of the light measurement well 13. As described above, as an example, the illumination direction N1 of the excitation light is aligned with the light receiving direction N2 of the emitted fluorescent light. The controller 40 computes analysis results based on data obtained by the light receiving section 52. Note that in cases in which a chemiluminescent substrate is employed as the enzyme substrate, there is no need to illuminate the light measurement well 13 with light using the light source section 51. The light receiving section 52 receives chemiluminescent light emitted in the light receiving direction labeled N2 through the light measurement well 13.

As illustrated as an example in Fig. 7, the light source section 51 includes the light emitting element 51 a, a colored glass filter 51 b, a beam splitter 51 c, an aperture 51d, a reference photodiode 51e, and an aperture 51f. The light receiving section 52 includes a light guide 52a and the light receiving element 52e.

The light emitting element 51a is used to illuminate the light measurement well 13 with excitation light. A light-emitting diode (LED) is employed as the light emitting element 51a. Examples of the LED include the NSHU591B (trade name), manufactured by Nichia Corporation, described above. As described above, the NSHU591B has a central wavelength of 365 nm. Note that specific examples of the light emitting element 51a other than LEDs include a laser diode, a xenon lamp, and a halogen lamp. Note that the light emitting element 51a corresponds to an example of a light source of technology disclosed herein.

The colored glass filter 51 b is used to select the wavelength of the excitation light. The colored glass filter 51b corresponds to an example of an excitation light wavelength selection filter of technology disclosed herein. Specific examples of the colored glass filter 51b include employing a U340 (trade name) manufactured by HOYA Corporation. The U340 is a filter employed in order to transmit only ultraviolet light, and allows light in the ultraviolet region to pass through while absorbing light in the visible light region. The thickness of the U340 is, for example, 2.5 mm. The excitation light wavelength selection filter is an optical component having absorption characteristics for predetermined wavelengths. Other than the colored glass filter 51b, specific examples of the excitation light wavelength selection filter include films that absorb predetermined wavelengths, colored aqueous solutions, and colored oils.

The beam splitter 51 c is used to split reference light from ultraviolet light that has passed through the colored glass filter 51b. The split-off ultraviolet passes through an opening 51 g provided in the aperture 51d, and is received by the reference photodiode 51e. The ultraviolet light received by the reference photodiode 51e is used to correct variation in the amount of light emitted from the light emitting element 51a.

The aperture 51 f includes an opening 51 h, and is a component for guiding, to the measurement solution L1 in the light measurement well 13, ultraviolet light passing through un-split by the beam splitter 51c. The ultraviolet light that has passed through the beam splitter 51c passes through the opening 51 h provided in the aperture 51f, and is illuminated onto the measurement solution L1 in the light measurement well 13.

The light guide 52a is a component for collecting fluorescent light or chemiluminescent light emitted from the bottom wall 13e of the light measurement well 13. For example, the light guide 52a is a hollow reflecting tube with an upper opening diameter of 13 mm, a lower opening diameter of 8 mm, and a height of 15 mm. An inner wall face 52d of the light guide 52a has a thin, metal film (not illustrated in the drawings) thereon, with an overcoat of a magnesium fluoride ($MgF_2$) layer over an aluminum (Al) layer. Note that the thin, metal film may include an overcoat of an SiO layer over the Al layer. Colored glass filters 52b and 52c are fitted into the upper opening and the lower opening of the light guide 52a. Specifically, ITY-425 (trade name) filters manufactured by Isuzu Glass, Ltd. are employed as the colored glass filters 52b and 52c. The cut-off wavelength of ITY 425 filters is 425 nm. Wavelengths of 425 nm or shorter are cut, and wavelengths of 425 nm or greater are allowed to pass through. The thickness of the ITY-425 filter is, for example, 1.1 mm. Note that configuration may be made in which a single ITY-425 filter with a thickness of 2.2 mm is disposed in the upper opening of the light guide 52a. The light guide 52a corresponds to an example of a light collecting member of technology disclosed herein. The colored glass filter 52b corresponds to an example of a measurement light wavelength selection filter of technology disclosed herein. The colored glass filter 52c also corresponds to an example of a measurement light wavelength selection filter of technology disclosed herein. Note that the measurement light wavelength selection filters are optical components having absorption characteristics for predetermined wavelengths. Other than the colored glass filters 52b and 52c, specific examples of the measurement light wavelength selection filters include films, colored aqueous solutions, and colored oils that absorb predetermined wavelengths.

The light receiving element 52e is a component for receiving fluorescent light or chemiluminescent light collected by the light guide 52a. The light receiving element 52e corresponds to an example of a detection element of technology disclosed herein. A photodiode (PD), for example, is employed as the light receiving element 52e. Specifically, for example, an S1337-1010BR (trade name) manufactured by Hamamatsu Photonics K.K. is employed as the PD. Other than PDs, specific examples of the light receiving element 52e include avalanche photodiodes, photomultipliers, CCD, and CMOS.

As illustrated as an example in Fig. 1, the placement tray 53 is a tray on which the analysis tool 1 and the biological sample rack 3 are placed. The placement tray 53 holds the analysis tool 1 such that upper openings of the reaction tube 11, the plural tubes 12, and the light measurement well 13 face upward. The placement tray 53 also holds the biological sample rack 3 such that an opening of the micro tube 30 faces upward.

As illustrated in Fig. 1, the horizontal drive section 9 moves the placement tray 53 in an X-axis direction (horizontal direction) orthogonal to a Z-axis direction. Namely, if needed, the horizontal drive section 9 moves the reaction tube 11, the plural tubes 12, and the light measurement well 13 on the analysis tool 1, and the biological sample rack 3, in a horizontal direction with respect to a nozzle 7, described below. The X-axis direction refers to a lateral direction. The horizontal drive section 9 includes a linear stage 9a and an X-axis motor 9b. The linear stage 9a includes a feed screw 90, a guide member 91, and a moving base 92. The moving base 92 engages with the feed screw 90 and the guide member 91 extending along the X-axis direction. The moving base 92 is joined to a bottom face 53a of the placement tray 53, and retains the placement tray 53. The X-axis motor 9b is fixed to a casing (not illustrated in the drawings) of the analysis device 2, and rotates the feed screw 90 in order to move the moving base 92 in the X-axis direction along the guide member 91. The X-axis motor 9b is connected to the controller 40, and is operated under the control of the controller 40.

As illustrated in Fig. 1, the dispensing section 6 includes a nozzle 7, a pump 70, a compression spring 73, and a raising/lowering drive section 8. The dispensing section 6 draws, moves, and dispenses the biological sample S, the liquid L, or the measurement solution L1 so as to transfer the biological sample S, the liquid L, or the measurement solution L1 between the biological sample rack 3, the reaction tube 11, the plural tubes 12, and the light measurement well 13. Moreover, the dispensing section 6 agitates the liquid L or the measurement solution L1 by repeatedly drawing and purging the liquid L or the measurement solution L1.

The nozzle 7 includes a nozzle body 72 and the pipette tip 71. The pipette tip 71 is detachably attached to the nozzle body 72. The nozzle 7 draws and purges the biological sample S, the liquid L, or the measurement solution L1 through a small hole 71 b in the pipette tip leading end 71 a of the pipette tip 71.

The pipette tip 71 is disposable, and employs a material such as propylene. The pipette tip leading end 71 a is flat, and has a circular shaped outer peripheral profile. The pipette tip leading end 71a has a diameter of 1.0 mm, for example. The small hole 71 b has a diameter of 0.5 mm, for example. The dispensing section 6 pierces the seal 11b of the reaction tube 11 and the seals 12i of the plural tubes 12 with the pipette tip leading end 71 a. Inside the pipette tip 71 there is a liquid holding section 71c that extends upward from the small hole 71 b of the pipette tip leading end 71 a, and that holds the biological sample S, the liquid L, or the measurement solution L1. The pipette tip 71 also includes an attachment portion 71 d for attachment to the nozzle body 72 above the liquid holding section 71c.

The nozzle 7 may be configured by the nozzle body 72 only, without employing the pipette tip 71 as a configuration element. For example, configuration may be made in which a nozzle body leading end portion 72a of the nozzle body 72 is used to draw or purge a liquid, such as the biological sample S, with the nozzle body leading end portion 72a being washed as required. In such a configuration, the nozzle body leading end portion 72a is configured with a tapered profile, and is configured so as to be capable of piercing the seals 11b and 12i.

The nozzle body 72 is made from stainless steel, for example. The nozzle body 72 is retained by a nozzle support 84, described later, such that the nozzle body 72 is surrounded by the nozzle support 84. The compression spring 73 is placed below the nozzle support 84. An upper end portion of the compression spring 73 abuts a lower end portion of the nozzle support 84. The nozzle body 72 passes through the inside of the compression spring 73, and includes a pipette tip mount 72b for mounting the pipette tip 71 at the nozzle body leading end portion 72a positioned at a lower end thereof. The pipette tip mount 72b is formed with a ring shaped recess, into which an O-ring 72c is fitted. The pipette tip mount 72b of the nozzle body 72 is inserted into the attachment portion 71d of the pipette tip 71, such that the two fit together. The pipette tip 71 can be removed from the nozzle body 72 by applying force to the pipette tip 71 in a direction to move the pipette tip 71 away from the nozzle body 72 along the axial direction of the pipette tip 71.

The nozzle body 72 is formed with a first ring shaped groove and a second ring shaped groove (not illustrated in the drawings) at approximately central positions. A first E-ring 72d and a second E-ring 72e are fitted into these respective ring shaped grooves. The first E-ring 72d is placed above the second E-ring 72e. The first E-ring 72d and the second E-ring 72e are placed with the nozzle support 84 and the compression spring 73 interposed between them. An upper face of the second E-ring 72e abuts a lower end portion of the compression spring 73. Accordingly, the compression spring 73 is retained so as not to drop down. A lower face of the first E-ring 72d abuts an upper face of the nozzle support 84. The nozzle 7 is thereby supported by the nozzle support 84 so as not to drop down. The nozzle body 72 has a hollow tube shape, and connects together the pipette tip 71 and the pump 70, described later.

The compression spring 73 is used to absorb shock by compressing and moving the nozzle 7 upward when, for whatever reason, the pipette tip 71 knocks against the micro tube 30 of the biological sample rack 3, the reaction tube 11, the plural tubes 12, or the light measurement well 13, for example.

The pump 70 is used to draw the biological sample S, the liquid L, or the measurement solution L1 into the pipette tip 71, and to purge the biological sample S, the liquid L, or the measurement solution L1 out from the pipette tip 71. The pump 70 is connected to a leading end 72f of the nozzle body 72 through a tube 74. The pump 70 is connected to the controller 40, and drawing and purging operations are controlled by the controller 40.

The raising/lowering drive section 8 is used to raise or lower the nozzle 7 in the Z-axis direction in a state in which the pipette tip leading end 71 a is facing downward. Note that the Z-axis direction indicates a vertical direction. The raising/lowering drive section 8 holds the pipette tip 71 through the nozzle body 72. The raising/lowering drive section 8 moves the nozzle 7 back and forth so as to raise and lower the pipette tip leading end 71 a in the Z-axis direction. The raising/lowering drive section 8 includes a linear stage 8a and the Z-axis motor 8b. The linear stage 8a includes a feed screw 80, a guide member 81 extending in the Z-axis direction, and a moving base 82. The moving base 82 includes a moving base body 83 and the nozzle support 84. The moving base body 83 engages with the feed screw 80 and the guide member 81 and retains the nozzle 7 through the nozzle support 84 such that the nozzle 7 is capable of vertical movement in a predetermined range. The nozzle support 84 is joined to and integrated together with the moving base body 83 by a joining portion 85. The Z-axis motor 8b is fixed to the casing (not illustrated in the drawings) of the analysis device 2, and the moving platform 82 is moved back and forth in the Z-axis direction along the guide member 81 by rotating the feed screw 80 of the linear stage 8a. The Z-axis motor 8b is connected to the controller 40, and is operated under the control of the controller 40.

As illustrated as an example in Fig. 1, the dispensing section 6 moves the pipette tip leading end 71a in a piercing direction (first direction), indicated by arrow N3, in order to pierce the seal 11b of the reaction tube 11 and the seals 12i of the plural tubes 12. Note that in Fig. 1, the piercing direction indicated by arrow N3 is, for example, a direction running along the Z-axis. The nozzle support 84 is sleeve shaped, and the nozzle body 72 passes through the inside of the nozzle support 84. As described above, the nozzle support 84 supports the nozzle body 72 so as to surround the periphery of the nozzle body 72. The nozzle support 84 is formed such that there is a gap 84a to the nozzle body 72 at the inside.

The nozzle 7 has play to the nozzle support 84 due to provision of the gap 84a. Accordingly, the nozzle 7 can undergo displacement with a degree of freedom in a direction (second direction) intersecting the piercing direction indicated by the arrow N3. Accordingly, the pipette tip leading end 71 a also undergoes displacement in this direction. As illustrated as an example in Fig. 1, the direction in which the nozzle 7 undergoes displacement is the direction indicated by the arrow N4 that intersects the piercing direction. The arrow N4 is not a single direction, and represents any direction that intersects the piercing direction labeled with the arrow N3.

If the micro tube 30 of the biological sample rack 3, the reaction tube 11, the plural tubes 12, or the light measurement well 13 has, for whatever reason, been set misaligned in the direction indicated by the arrow N4, the pipette tip leading end 71 a slides in a direction to eliminate the misalignment (along the direction indicated by the arrow N4: in the opposite direction to the misalignment). This thereby enables a collision between the pipette tip leading end 71a and the micro tube 30 of the biological sample rack 3, the reaction tube 11, the plural tubes 12, or the light measurement well 13, to be avoided. This further enables damage to these members to be avoided.

Next, explanation follows regarding an example of operations of the analysis device 2 to transfer the liquid L and the measurement solution L1 between the tubes of the analysis tool 1, and to measure the measurement solution L1 with the detection section 5, in the analysis system AS, with reference to Fig. 1, Fig. 7, and Fig. 8. Transfer of the liquid L and the measurement solution L1 is executed by controlling operations of the raising/lowering drive section 8 and the horizontal drive section 9 by the controller 40 illustrated in Fig. 1.

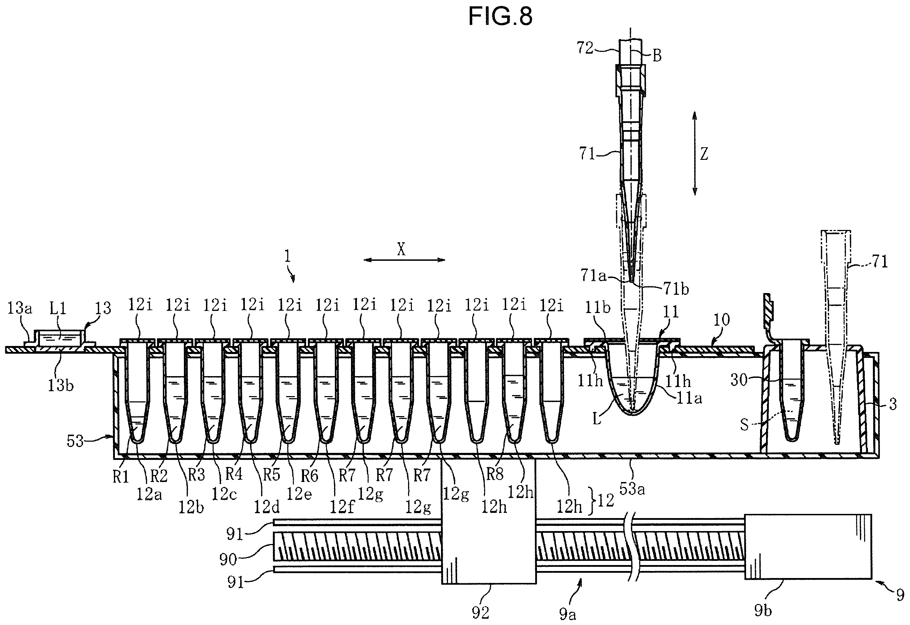

As illustrated as an example in Fig. 8, first, the biological sample rack 3 is moved along the X-axis, and placed at a reference position B. The nozzle body 72 is moved downward, and the pipette tip 71 is mounted on the nozzle body 72. Then, the pipette tip 71 is placed at a predetermined height at the reference position B. Next, a buffer wash solution tube 12g is moved to the reference position B. The pipette tip 71 is moved downward toward the buffer wash solution tube 12g, the pump 70 is driven so as to draw a predetermined amount of the buffer wash solution R7 from the buffer wash solution tube 12g, and the pipette tip 71 is then moved upward. The reaction tube 11 is then moved to the reference position B. The pipette tip 71 is moved downward and purges the buffer wash solution R7 into the reaction tube 11. After a predetermined duration has elapsed, the pipette tip 71 draws the buffer wash solution R7 from the reaction tube 11 and moves upward. Then, a waste solution tube 12h is moved to the reference position B. The pipette tip 71 is moved downward toward the waste solution tube 12h, and purges the buffer wash solution R7 as waste solution R8.

Next, the biological sample dilution tube 12b is moved to the reference position B. The pipette tip 71 is moved downward, draws a predetermined amount of the biological sample dilution solution R2 from the biological sample dilution tube 12b, and is moved upward. Then, a biological sample dilution tube 12a is moved to the reference position B. The pipette tip 71 is moved downward toward the biological sample dilution tube 12a, and after purging the biological sample dilution solution R2 therein, is moved upward. Next, the biological sample rack 3 is moved to the reference position B. The pipette tip 71 is moved downward, draws a predetermined amount of the biological sample S from the micro tube 30, and is then moved upward. Then, the biological sample dilution tube 12a is moved to the reference position B. The pipette tip 71 is moved downward toward the biological sample dilution tube 12a, and purges the biological sample S. Then, the pipette tip 71 draws and purges (discharges) so as to mix the biological sample S and the biological sample dilution solution R2 together, and so as to adjust the mixture solution R1. The biological sample S is diluted by a predetermined dilution factor using this method.

Next, the pipette tip 71 draws a predetermined amount of the mixture solution R1 from the biological sample dilution tube 12a. Then, the reaction tube 11 is moved to the reference position B. The pipette tip 71 is moved downward toward the reaction tube 11, purges the mixture solution R1, and is moved upward. Then, the reaction tube 11 is incubated at a predetermined temperature for a predetermined duration. Accordingly, the specified component in the mixture solution R1 thus binds to the immobilized antibody in the reaction tube 11. Then, the pipette tip 71 is moved downward, draws the mixture solution R1 from the reaction tube 11, and is moved upward. Then, the waste solution tubes 12h is moved to the reference position B. The pipette tip 71 is moved downward, discards the mixture solution R1 into the waste solution tube 12h as waste solution R8, and is then moved upward. Next, a buffer wash solution tubes 12g is moved to the reference position B. The pipette tip 71 is moved downward toward the buffer wash solution tube 12g, draws a predetermined amount of the buffer wash solution R7, and is moved upward. Next, the reaction tube 11 is moved to the reference position B. The pipette tip 71 is moved downward toward the reaction tube 11, and purges the buffer wash solution R7. The pipette tip 71 then quickly draws the buffer wash solution R7, and is moved upward. Then, the waste solution tube 12h is moved to the reference position B. The pipette tip 71 is moved downward toward the waste solution tube 12h, and discards the buffer wash solution R7 as waste solution R8. The pipette tip 71 repeats this washing operation a predetermined number of times.

Next, the primary antibody solution tube 12c is moved to the reference position B. The pipette tip 71 is moved downward toward the primary antibody solution tube 12c, draws a predetermined amount of the primary antibody solution R3, and is moved upward. Then the reaction tube 11 is moved to the reference position B. The pipette tip 71 is moved downward toward the reaction tube 11, and purges the primary antibody solution R3. The primary antibody accordingly binds with the specified component captured by the immobilized antibody 11 f. After incubating the reaction tube 11 for a predetermined duration, the pipette tip 71 draws the primary antibody solution R3 from the reaction tube 11, and is then moved upward. Then the waste solution tube 12h is moved to the reference position B. The pipette tip 71 is moved downward and discards the primary antibody solution R3 as waste solution R8 in the waste solution tube 12h. Next, the buffer wash solution tube 12g is moved to the reference position B. The pipette tip 71 is moved downward toward the buffer wash solution tube 12g, draws a predetermined amount of the buffer wash solution R7, and is moved upward. Then, the reaction tube 11 is moved to the reference position B. The pipette tip 71 is moved downward toward the reaction tube 11, and purges the buffer wash solution R7. Then, the pipette tip 71 quickly draws the buffer wash solution R7, and is moved upward. Then, the waste solution tube 12h is moved to the reference position B. The pipette tip 71 is moved downward toward the waste solution tube 12h, discards the buffer wash solution R7 as waste solution R8, and is moved upward. The pipette tip 71 repeats this washing operation a predetermined number of times.

Next, the secondary antibody solution tube 12d is moved to the reference position B. The pipette tip 71 is moved downward toward the secondary antibody solution tube 12d, draws a specific amount of the secondary antibody solution R4, and is moved upward. Then, the reaction tube 11 is moved to the reference position B. The pipette tip 71 is moved downward toward the reaction tube 11, and purges the secondary antibody solution R4. The secondary antibody accordingly binds to the primary antibody bound to the specified component. After incubating the reaction tube 11 for a specific duration, the pipette tip 71 draws the secondary antibody solution R4 from the reaction tube 11, and is then moved upward. Then, the waste solution tube 12h is moved to the reference position B. The pipette tip 71 is moved downward toward the waste solution tube 12h, discards the secondary antibody solution R4 as waste solution R8, and is then moved upward. Next, the buffer wash solution tube 12g is moved to the reference position B. The pipette tip 71 is moved downward, draws a specific amount of the buffer wash solution R7 from the buffer wash solution tube 12g, and is moved upward. Then, the reaction tube 11 is moved to the reference position B. The pipette tip 71 is moved downward toward the reaction tube 11, and purges the buffer wash solution R7. Then the pipette tip 71 quickly draws the buffer wash solution R7, and is moved upward. Then, the waste solution tube 12h is moved to the reference position B. The pipette tip 71 is moved downward toward the waste solution tube 12h, discards the buffer wash solution R7 as waste solution R8, and is moved upward. The pipette tip 71 repeats this washing operation a predetermined number of times.

Next, the enzyme substrate solution tube 12e is moved to the reference position B. The pipette tip 71 is moved downward toward the substrate solution tube 12e, and after drawing a predetermined amount of the enzyme substrate solution R5, is moved upward. Then, the reaction tube 11 is moved to the reference position B. The pipette tip 71 is moved downward toward the reaction tube 11, and purges the enzyme substrate solution R5. The marker enzyme of the secondary antibody accordingly reacts with the enzyme substrate contained in the enzyme substrate solution R5. After incubating the reaction tube 11 for a predetermined duration, the reaction-stop solution tube 12f is moved to the reference position B. The pipette tip 71 is moved downward toward the reaction-stop solution tube 12f, draws a predetermined amount of the reaction stop solution R6, and is then moved upward. Then, the reaction tube 11 is moved to the reference position B. The pipette tip 71 is moved downward toward the reaction tube 11, and purges the reaction stop solution R6. The marker enzyme is thereby denatured, such that the enzyme reaction stops, and the measurement solution L1 is generated. Then, the pipette tip 71 draws the measurement solution L1 from the reaction tube 11, and is moved upward. Then, the light measurement well 13 is moved to the reference position B. The pipette tip 71 is moved downward toward the light measurement well 13, and transfers the measurement solution L1 into the light measurement well 13.

Next, the light measurement well 13 is moved to the position of the optical system 50. The optical system 50 measures the measurement light emitted from the light measurement well 13.

Note that when the measurement light is measured by the optical system 50, the amount of the measurement light, such as fluorescent light and/or chemiluminescent light, emitted from the measurement solution L1 sometimes enters a state of excess as the concentration of the measurement solution L1 increases. In such cases, for example, a first behavior pattern and a second behavior pattern are conceivable behavior patterns of the output values of the light receiving element 52e with respect to the concentration of the measurement solution L1.

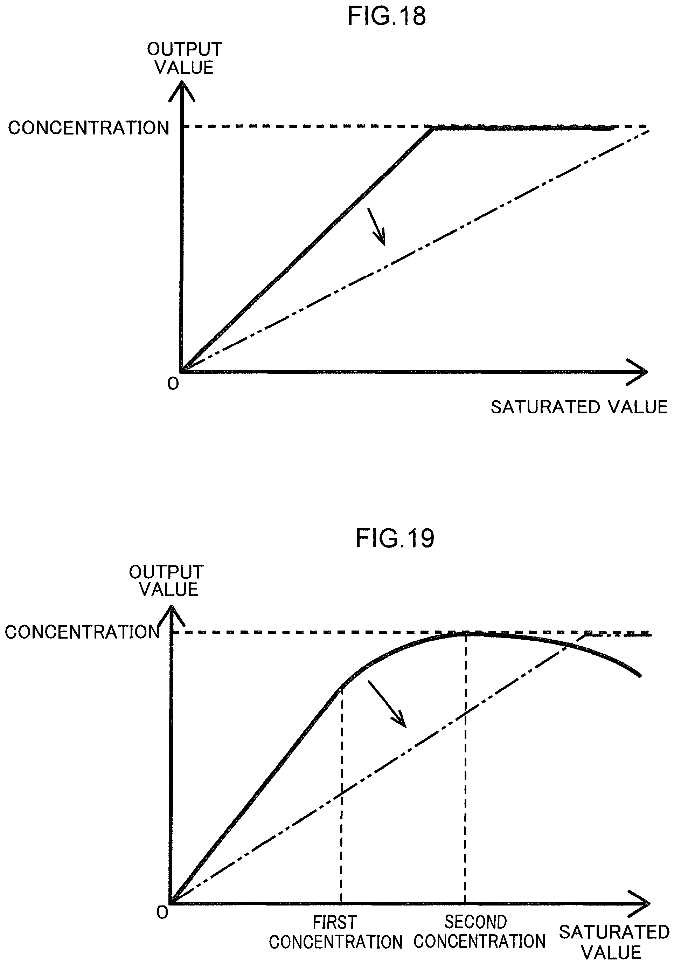

In the first behavior pattern, as illustrated as an example in Fig. 18, there is a linear increase in the output values of the light receiving element 52e as the concentration of the measurement solution L1 increases, reaching a saturation value at a predetermined concentration (for example, 256) or greater.

In the second behavior pattern, as illustrated as an example in Fig. 19, there is a magnitude relationship "first concentration < second concentration" for the concentration of the measurement solution L1. In such cases, in the second behavior pattern, as illustrated in the example of Fig. 19, in a concentration range of the first concentration or lower, the output value of the light receiving element 52e increases linearly with increasing concentration of the measurement solution L1, until reaching the first concentration. Moreover, in the second behavior pattern, increase in the output value of the light receiving element 52e gradual decreases with an exponential function between the first concentration and the second concentration, before reaching the saturation value at the second concentration. Moreover, in the second behavior pattern, in a concentration range higher than the second concentration, the output value of the light receiving element 52e gradual decreases with an exponential function as the concentration increases. When there is a gradual exponential decrease in the output values of the light receiving element 52e in this manner, the amount of light received by the light receiving element 52e becomes insufficient for the light emitted from the measurement solution L1.

Accordingly, in the analysis device 2, when the output value of the light receiving element 52e becomes saturated, the amount of the measurement solution L1 is decreased using the pipette tip 71, shortening the cell length D1. Measurement is then retaken after this has been completed. The first behavior pattern and the second behavior pattern are improved as a result. Namely, as illustrated as an example by the double-dotted dashed line in Fig. 18, the first behavior pattern is improved such that the output value of the light receiving element 52e does not become saturated at a predetermined concentration, and maintains its linearity. Moreover, as illustrated as an example by the double-dotted dashed lines in Fig. 19, the second behavior pattern is also improved, such that the linearity of the output value of the light receiving element 52e is maintained even in a concentration range in excess of the first concentration, and the output value of the light receiving element 52e increases linearly even in a concentration range higher than the second concentration.

Explanation follows regarding an example of measurement processing, illustrated in Fig. 20, as predetermined processing to implement the re-measuring of the measurement light.

The measurement processing illustrated as an example in Fig. 20 is executed by the CPU 100 as the CPU 100 follows the measurement program 106 (see Fig. 17).

In the measurement processing illustrated in Fig. 20, at step 200, the CPU 100 starts measurement of the measurement light by the optical system 50. Processing then processing transitions to step 202.

At step 202, the CPU 100 determines whether or not the output value of the light receiving element 52e is greater than "0". If the output value of the light receiving element 52e is greater than "0" at step 202, determination is affirmative, and processing transitions to step 204. If the output value of the light receiving element 52e is "0" at step 202, determination is negative, and processing transitions to step 206.

At step 204, the CPU 100 determines whether or not the output value of the light receiving element 52e is in a non-saturated state. Note that the non-saturated state refers, for example, to the output value (digital output value) of the light receiving element 52e being less than "256".

If the output value of the light receiving element 52e is in a non-saturated state at step 204, determination is affirmative, and processing transitions to step 206. If the output value of the light receiving element 52e is in a saturated state at step 204, determination is negative, and processing transitions to step 208.

At step 206, the CPU 100 determines whether or not a condition for ending measurement of the measurement light by the optical system 50 (referred to below as an "end condition") has been satisfied. A specific example of the end condition is a condition of the CPU 100 obtaining an output value greater than "0" continuously over a predetermined duration (for example, 1 second) or greater as the output value of the light receiving element 52e. Another specific example of the end condition is a condition of input through the input section 42 of a command to forcibly end the current measurement processing.

If the end condition has not been satisfied at step 206, determination is negative, and processing transitions to step 202. If the end condition has been satisfied at step 206, determination is affirmative, and the present measurement processing is ended.

At step 208, the CPU 100 ends the measurement of the measurement light by the optical system 50. Processing then transitions to step 210.

At step 210, the CPU 100 derives a reduction amount based on the time taken from receiving the measurement light with the light receiving element 52e until the output value of the light receiving element 52e reaches the saturated state. Note that "time taken from receiving the measurement light with the light receiving element 52e until the output value of the light receiving element 52e reaches the saturated state" refers, for example, to the time taken from affirmative determination being made at step 202 until the present moment. Moreover, the "reduction amount" refers to the amount by which to reduce the measurement solution L1. In the following explanation, for convenience, the "time taken from receiving the measurement light with the light receiving element 52e until the output value of the light receiving element 52e reaches the saturated state" is referred to simply as "the time taken to reach the saturated state".

The reduction amount is derived using a reduction amount derivation computation formula. The reduction amount derivation computation formula is a computation formula using the time taken to reach the saturated state as an independent variable, and the reduction amount as a dependent variable. Note that the reduction amount employed as the dependent variable is a value obtained in advance through actual testing and/or computer simulation as a reduction amount able to realize the behavior illustrated as an example by the double-dotted dashed lines in Fig. 18 and Fig. 19 when the measurement light is re-measured.

Note that explanation is given regarding an example of a case in which the reduction amount is derived using the reduction amount derivation computation formula. However, the technology disclosed herein is not limited thereto. For example, configuration may be made in which the reduction amount is derived using a reduction amount derivation table in which times taken to reach the saturated state and reduction amounts are associated with each other.

Moreover, explanation is given regarding an example of a case in which a one-to-one relationship between the time taken to reach the saturated state and the reduction amount is defined using the reduction amount derivation computation formula. However, the technology disclosed herein is not limited thereto. For example, configuration may be made in which the reduction amount is derived using a reduction amount derivation computation formula including a dependent variable, a first independent variable, and a second independent variable. In such cases, the dependent variable denotes the reduction amount. The first independent variable denotes the time taken to reach the saturated state. The second independent variable denotes the rate of rise in which the output value of the light receiving element 52e during an initial light emission period of the measurement solution L1, namely a predetermined period designated as an initial reaction period of the measurement solution L1. Note that the reduction amount may also be derived using a reduction amount derivation table in which times taken to reach the saturated state, rate of rise in the output value in the initial light emission period of the measurement light, and reduction amounts are associated with each other.

Moreover, the reduction amount may be derived using a reduction amount derivation computation formula defined with the time taken to reach the saturated state not used as an independent variable, the rate of rise of the output value of the light receiving element 52e during a period predetermined as an initial reaction period of the measurement solution L1 is used as an independent variable, and the reduction amount as a dependent variable. Note that the reduction amount may also be derived using a reduction amount derivation table in which speeds of increase of the output value during the initial light emission period of the measurement light and reduction amounts are associated with each other.

At the next step 212, the CPU 100 controls the dispensing section 6 so as to reduce the measurement solution L1 by the reduction amount derived by the processing of step 210. Processing then transitions to step 200.

In cases in which the enzyme substrate employed is a fluorogenic substrate (for example, 4-MUP), as illustrated as an example in Fig. 7, the light emitting element 51a illuminates the measurement solution L1 directly through the upper opening 13c along the illumination direction N1 with excitation light of a predetermined wavelength (for example, 365 nm). The light receiving section 52 receives fluorescent light (for example, 450 nm) emitted through the lower face 13h of the bottom wall 13e in the light receiving direction N2. In cases in which the substrate employed is a chemiluminescent substrate, the light receiving section 52 receives chemiluminescent light emitted through the lower face 13h of the bottom wall 13e in the light receiving direction N2. Data output by the light receiving element 52e of the light receiving section 52 is sent to the controller 40 illustrated in Fig. 1. The controller 40 computes analysis results based on this data.

According to the present embodiment, the measurement solution holder 13d of the light measurement well 13 has a profile flattened in the light receiving direction N2 of the light receiving element 52e. Since the cell length is short, the excitation light is not readily absorbed by the measurement solution L1 containing the fluorogenic substrate. Accordingly, intense excitation light hits the whole of the measurement solution L1. Moreover, the viewing angle from the uppermost portion of the measurement solution L1 to the light receiving element 52e becomes wider due to the shortened cell length. The light collecting efficiency is thereby improved, enabling high precision analysis of the specified component in the biological sample S. Moreover, since the cell length can be shortened, the amounts of the reagents can be suppressed. This thereby enables a reduction in manufacturing costs of the analysis tool 1.

The analysis tool 1 includes the reaction tube 11 for immobilizing the antibody or antigen for the specified component contained in the biological sample S, and for generating the measurement solution L1. The analysis tool 1 is thereby capable of high precision analysis of the specified component in the biological sample S.

In the analysis tool 1, in cases in which the measurement light is fluorescent light, the excitation light used to cause the fluorescent light to be emitted is illuminated directly into the measurement solution L1 through the upper opening 13c. This thereby enables intense excitation light to hit the measurement solution L1 . This thereby enables the light collecting efficiency to be improved, enabling high precision analysis of the specified component in the biological sample S.

The analysis device 2 includes the colored glass filters 51b, 52b, 52c serving as measurement light wavelength selection filters or excitation light wavelength selection filters. Employing the colored glass filters 51b, 52b, 52c enables clearer signals of the measurement light. This thereby enables light collecting efficiency to be improved, enabling high precision analysis of the specified component in the biological sample S.

The detection section 5 of the analysis device 2 is hollow, and includes the light guide having a thin, metal film at the inner peripheral face. This thereby enables larger signals of the measurement light. This thereby enables light collecting efficiency to be improved, enabling high precision analysis of the specified component in the biological sample S.

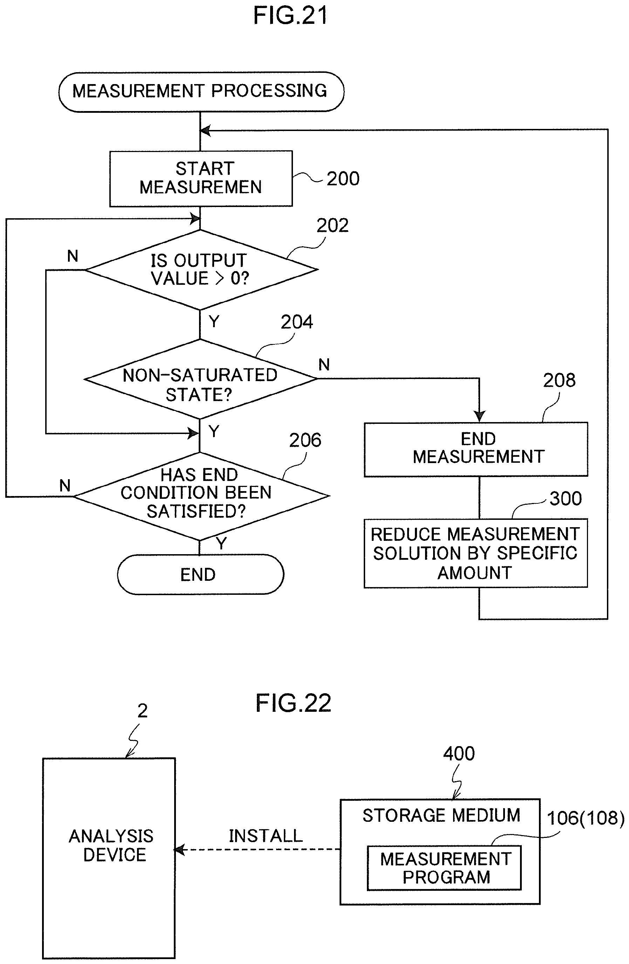

Note that in the first embodiment described above, explanation has been given regarding a case in which the reduction amount of the measurement solution L1 is derived by the processing at step 210 by executing the measurement processing illustrated in Fig. 20. However, this is merely an example. For example, configuration may be made in which the measurement processing illustrated in Fig. 21 is executed by the CPU 100.

As illustrated as an example in Fig. 17, a measurement program 108 is stored in the ROM 104. The CPU 100 reads the measurement program 108 from the ROM 104 and the CPU 100 executes the measurement processing illustrated in Fig. 21 by following the read measurement program 108. Note that the measurement processing illustrated in Fig. 21 differs from the measurement processing illustrated in Fig. 20 in the point that it includes a step 300 in the place of step 210 and step 212. Accordingly, explanation of the measurement processing illustrated in Fig. 21 deals with only the elements differing from those of the measurement processing illustrated in Fig. 20.

In the measurement processing illustrated in Fig. 21, at step 300, the CPU 100 reduces the measurement solution L1 by a predetermined amount, and then processing transitions to step 200. Note that the predetermined amount is, for example, an amount corresponding to 5% of the current amount of the measurement solution L1. Accordingly, the processing of step 300 is repeated if the output value of the light receiving element 52e reaches the saturated state again, even after the measurement solution L1 has been reduced by executing the processing of the step 300 a single time.

Explanation has been given regarding an example of cases in which the measurement programs 106, 108 (referred to as the "measurement program" without reference numerals below) are read from the ROM 104. However, the measurement program does not necessarily need to be stored in the ROM 104 initially. For example, as illustrated in Fig. 22, the measurement program may first be stored on any appropriate portable storage medium 400 such as a Solid State Drive (SSD), Universal Serial Bus (USB) memory, or a Compact Disk Read Only Memory (CD-ROM). In such cases, the measurement program on the storage medium 400 is installed in the analysis device 2, and the installed measurement program is executed by the CPU 100.

Moreover, configuration may be made in which the measurement program is stored in a storage section such as another computer, or server device, connected to the analysis device 2 through a communications network (not illustrated in the drawings), and the measurement program is downloaded in response to a request from the analysis device 2. In such cases, the downloaded program is executed by the CPU 100.

The measurement processing described above (see Fig. 20 and Fig. 21) are merely examples thereof. Obviously, unnecessary steps may be removed, and new steps may be added, and the processing sequence may be changed around, within a range not departing from the basic spirit. Moreover, the respective processing included in the measurement processing may be implemented solely by a hardware configuration such as a Field-Programmable Gate Array (FPGA) or an Application Specific Integrated Circuit (ASIC), or may be implemented using a combination of software configurations and hardware configurations employing a computer.

Second Embodiment

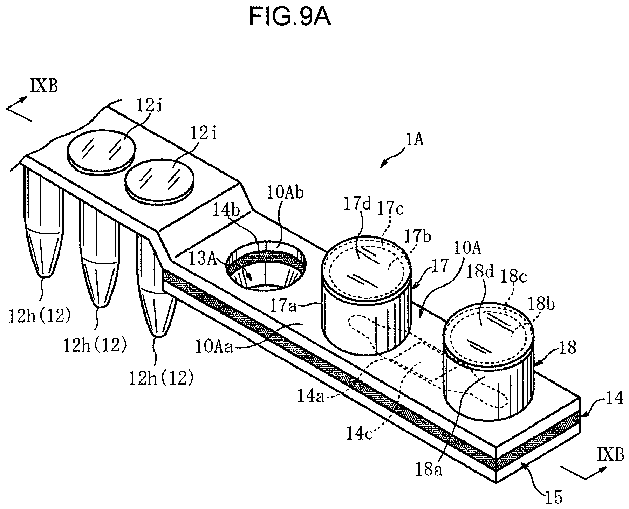

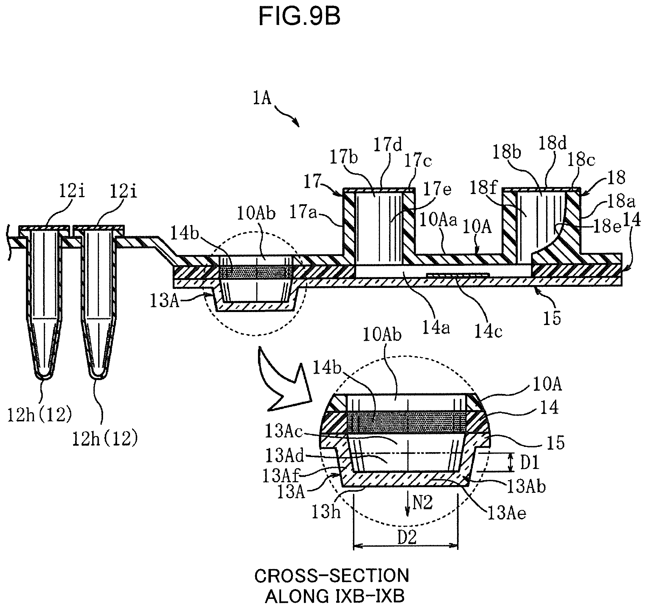

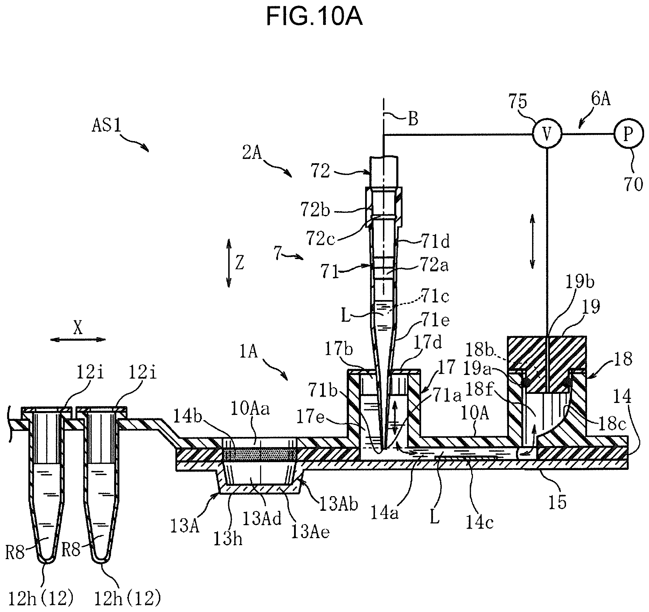

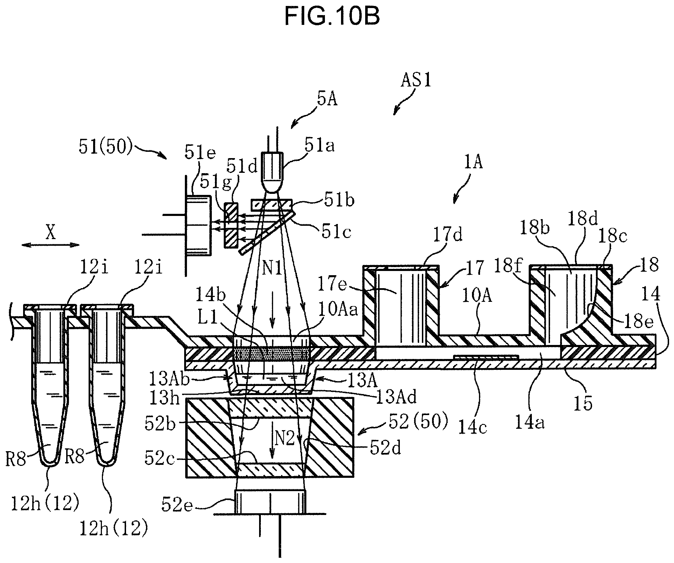

Next, explanation follows regarding an analysis tool 1A according to a second embodiment of technology disclosed herein, with reference to Fig. 9A, Fig. 9B, Fig. 10A, and Fig. 10B. In the analysis tool 1A, configuration elements that have functions the same as or similar to that of configuration elements of the analysis tool 1 of the first embodiment are allocated the same reference numerals. Detailed explanation of these configuration elements is omitted. The analysis tool 1A is employed with an analysis device 2A and an analysis system AS1. The analysis device 2A and the analysis system AS1 are substantially the same as the analysis device 2 and the analysis system AS of the first embodiment, with the exception of the configurations described below, and so detailed explanation thereof is omitted.

As illustrated as an example in Fig. 9A and Fig. 9B, the analysis tool 1A includes an upper base plate 10A, an immobilization plate 15, and an adhesive layer 14. The upper base plate 10A corresponds to an example of a first base plate of technology disclosed herein. The upper base plate 10A is, for example, a polypropylene extrusion molded component, and, similarly to the analysis tool 1, is mounted with, or is integrally molded with, plural tubes 12. An upper face 10Aa of the upper base plate 10A is provided with a tube shaped protrusion 17. The tube shaped protrusion 17 includes a side wall 17a and a first opening 17b. A seal 17d for closing off the first opening 17b is adhered to an upper end portion 17c of the side wall 17a. The first opening 17b is a location where the pipette tip 71 is inserted. The seal 17d is broken by the pipette tip leading end 71 a when the pipette tip 71 is inserted.

The upper face 10Aa of the upper base plate 10A is further provided with a tube shaped protrusion 18. The tube shaped protrusion 18 includes a side wall 18a and a second opening 18b. A seal 18d for closing off the second opening 18b is adhered to an upper end portion 18c of the side wall 18a. The second opening 18b is a location where a drawing/discharging nozzle 19, described later, is inserted. A portion of an inner face 18e of the side wall 18a is formed with a downward sloping curved shape.

The immobilization plate 15 includes immobilized antibody 14c and a light measurement well 13A. The immobilization plate 15 is, for example, a polystyrene extrusion molded component. A GPPS grade having low autofluorescence emission strength is low is employed as the material for the polystyrene immobilization plate 15. Specifically, HF77, HH102, and SGP10 (trade names), manufactured by PS Japan Corporation, are preferable examples of the molding material. Note that the immobilization plate 15 corresponds to an example of a second base plate of technology disclosed herein.

The material of the immobilization plate 15 is not limited to polystyrene (PS), and transparent or translucent resins may also be employed, such as poly (methyl methacrylate) (PMMA), cyclo-olefin polymer (COP), polycarbonate (PC), low density polyethylene (LDPE), polylactic acid (PLA), polydimethylsiloxane (PDMS), and polypropylene (PP). When a material is employed that antibodies do not readily physically attach to, carboxyl groups or amino groups are introduced to the surface of the immobilization plate 15 after performing vacuum ultraviolet (VUV) processing, plasma processing, chemical processing, or the like. The antigen or antibody is immobilized by covalent bonding to these functional groups. Alternatively, the antibody may be immobilized after applying a coating such as a self-assembled monolayer (SAM).

The light measurement well 13A is formed integrally molded to the immobilization plate 15. The light measurement well 13A includes a well body 13Ab. The well body 13Ab includes an upper opening 13Ac and a measurement solution holder 13Ad. The upper opening 13Ac corresponds to an example of an opening of technology disclosed herein. Openings 10Ab and 14b, respectively provided at the upper base plate 10A and the adhesive layer 14, are provided so as to be superimposed on each other above the upper opening 13Ac, and the pipette tip 71 is inserted through the opening 10Ab of the upper base plate 10A. The shape of the measurement solution holder 13Ad is formed similarly to that of the measurement solution holder 13d of the light measurement well 13 of the analysis tool 1 of the first embodiment. The measurement solution L1 is held in the measurement solution holder 13Ad of the well body 13Ab. The measurement solution holder 13Ad is formed so as to be surrounded by a side wall 13Af and a bottom wall 13Ae. The thickness of the side wall 13Af and the bottom wall 13Ae of the light measurement well 13A (at locations hit by excitation light) is formed at 1.0 mm or less. Light scattering and autofluorescence are preferably suppressed as much as possible by, for example, locally forming locations with a thickness of 0.5 mm.

As illustrated in Fig. 9B, the shape of the measurement solution holder 13Ad is a profile flattened in the light receiving direction N2. In the light measurement well 13A, the shape of the measurement solution holder 13Ad is, for example, determined by the height (depth) of the measurement solution L1 dispensed into the well body 13Ab, and by the internal diameter of the well body 13Ab. The height (depth) of the measurement solution holder 13Ad is the cell length D1 of the light measurement well 13A. Specifically, the cell length D1 of the light measurement well 13A is determined by the height (depth), in the crosswise direction of the cross-section of the flattened profile, of the measurement solution L1 dispensed into the measurement solution holder 13Ad. Moreover, the internal diameter of the well body 13Ab is a diameter D2 of the measurement solution holder 13Ad. Note that the shapes of the well body 13Ab and the light measurement well 13A may be configured as overall flattened profiles matching the shape of the measurement solution holder 13Ad. In such cases, the lower face 13h of the bottom wall 13Ae would be a flattened face. This flattened face is not limited to being a flat horizontal face, and may be a gently undulating face, or a curved face. Note that the lower face 13h corresponds to an example of an emission portion of technology disclosed herein.

The cell length D1 of the light measurement well 13A is, for example, 3.0 mm or less, 1.5 mm to 3.0 mm, 1.9 mm to 2.5 mm, 2.5 mm, 2.0 mm, 1.5 mm to 2.0 mm, 1.9 mm to 2.0 mm, 2.0 mm to 2.5 mm, 2.0 mm to 3.0 mm, or 2.0 mm or less. The diameter D2 of the light measurement well 13A is, for example, 8.0 mm or less, 8.0 mm to 11.3 mm, 8.8 mm to 10.0 mm, 9.8 mm, 10.0 mm, 9.8 mm to 10.0 mm, 8.8 mm to 9.8 mm, 9.8 mm to 10.0 mm, 8.8 mm to 9.8 mm, or 3.0 mm to 5.0 mm. The shape of the measurement solution holder 13Ad when 150 μL of the measurement solution L1 has been dispensed into the light measurement well 13A is, for example, φ 9.8 × 2.0 mm. In such cases, the shape of the light measurement well 13A is φ 9.8 or greater, with a height (depth) of 2 mm or greater.

The analysis tool 1A may be configured such that the cell length D1 can be changed by changing the amount of the measurement solution L1 dispensed into the measurement solution holder 13Ad. By configuring the light measurement well 13A in this manner, when output values of the light receiving element 52e become saturated, the analysis device 2, described later, re-performs measurement after reducing the amount of the measurement solution L1 and shortening the cell length D 1.

The adhesive layer 14 is a member for adhering the upper base plate 10A and the immobilization plate 15 together. For example, the adhesive layer 14 is formed using double-sided tape, and includes a reaction flow path 14a. The reaction flow path 14a is formed by punching the adhesive layer 14. The reaction flow path 14a links together an inside 17e of the tube shaped protrusion 17 and an inside 18f of the tube shaped protrusion 18. Antigen-antibody reactions and enzyme reactions take place inside the reaction flow path 14a. The liquid L and the measurement solution L1 are moved back and forth inside the reaction flow path 14a by the drawing/discharging nozzle 19, described later. The size of the reaction flow path 14a is, for example, set so as to approximate to a cuboid of length 30 mm × width 5 mm × height (depth) 0.15 mm. In such a configuration, the flow path capacity of the reaction flow path 14a is 22.5 μL. The immobilization surface area of immobilized antibody 14c is, for example, 150 mm$^2$. Considering the reactivity between the biological sample S and the immobilized antibody 14c, the reactivity of the marker enzyme, and the fluorescent light emission strength, the amount of solution moved back and forth in the reaction flow path 14a is, for example, 200 μL, and the amount of the measurement solution L1 transferred to the light measurement well 13A after the enzyme reaction is, for example, set to 150 μL. The adhesive layer 14 is colored black. The adhesive layer 14 acts as a mask surrounding and following the profile of the light measurement well 13A, which is integrally formed at the immobilization plate 15, thereby blocking excitation light illuminated into the light measurement well 13A.

As illustrated as an example in Fig. 10A, the analysis device 2A configuring the analysis system AS1 includes a dispensing section 6A. The dispensing section 6A includes the drawing/discharging nozzle 19 and a switching valve 75. The switching valve 75 switches coupling of the pump 70 between the nozzle 7 and an air hole 19b in the drawing/discharging nozzle 19. The drawing/discharging nozzle 19 is fitted into the second opening 18b through an O-ring 19a. The drawing/discharging nozzle 19 includes the air hole 19b connecting between the interior and the exterior of the analysis tool 1A. The pump 70 is, for example, a syringe pump. After using the pipette tip 71 to dispense the liquid L into the inside 17e of the tube shaped protrusion 17, the controller 40 switches to the drawing/discharging nozzle 19, and moves the liquid L back and forth through the reaction flow path 14a by drawing and then discharging air, as indicated by the arrows. Note that movement back and forth of the liquid L may be performed by a combination of drawing and discharging the liquid L with the pipette tip 71, and drawing and discharging air with the drawing/discharging nozzle 19. The measurement solution L1 finally obtained by swapping the liquid L in sequence is transferred to the light measurement well 13A using the pipette tip 71.

As illustrated as an example in Fig. 10B, similarly to in the first embodiment, the measurement solution L1 is measured using the detection section 5. Similarly to in the first embodiment, data output from the light receiving element 52e of the light receiving section 52 is sent to the controller 40. The controller 40 computes analysis results based on this data.

In the present embodiment, the measurement solution holder 13Ad of the light measurement well 13A has a profile flattened in the light receiving direction of the light receiving element 52e. This thereby enables similar advantageous effects to those of the first embodiment to be achieved.

The reaction flow path 14a of the analysis tool 1A immobilizes the antibody or antigen for the specified component contained in the biological sample S, and generates the measurement solution L1. The analysis tool 1A is thereby capable of high precision analysis of the specified component in the biological sample S.

The analysis device 2A is configured to move the liquid L and the measurement solution L1 back and forth in the reaction flow path 14a using the drawing/discharging nozzle 19. This thereby enables the uniformity of the measurement reaction, and washing performance, to be improved, thereby enabling high precision analysis of the specified component in the biological sample S. In other respects, the analysis device 2A is capable of similar advantageous effects to those of the first embodiment.

Third Embodiment

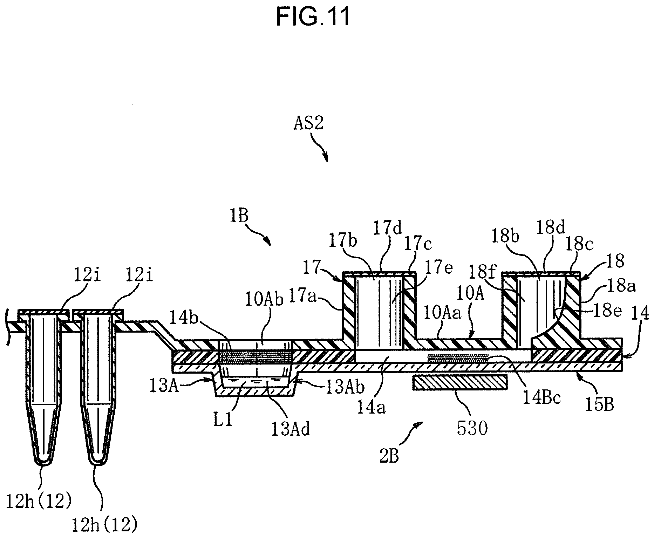

Next, explanation follows regarding an analysis tool 1B according to a third embodiment of technology disclosed herein, with reference to Fig. 11. In the analysis tool 1B, configuration elements that have functions the same as or similar to configuration elements of the analysis tool 1A of the second embodiment are allocated the same reference numerals. Detailed explanation of these configuration elements is omitted. The analysis tool 1B is employed with an analysis device 2B and an analysis system AS2. The analysis device 2B and the analysis system AS2 are substantially the same as the analysis device 2A and the analysis system AS1 of the second embodiment, with the exception of the configurations described below, and so detailed explanation thereof is omitted.

The analysis tool 1B differs from the analysis tool 1A in the point that the analysis tool 1B includes a plate 15B. The plate 15B corresponds to an example of a second base plate of technology disclosed herein. The light measurement well 13A is formed integrally molded to the plate 15B. In the analysis tool 1B, the antibody is not directly immobilized on the plate 15B; immobilized magnetic particles 14Bc are disposed on the plate 15B inside the reaction flow path 14a. In the analysis device 2B, a magnet 530 is placed below the analysis tool 1B. The magnet 530 keeps the immobilized magnetic particles 14Bc within the reaction flow path 14a by magnetic force as the measurement solution L1 is moved into the light measurement well 13A, and is used to prevent the immobilized magnetic particles 14Bc from moving into the light measurement well 13A. Specific examples of the magnet 530 include an electromagnet or a permanent magnet. Note that a solution of antibody-sensitized magnetic beads may be prepared as a separate reagent rather than disposing the immobilized magnetic particles 14Bc from the outset.

The analysis tool 1B includes the immobilized magnetic particles 14Bc inside the reaction flow path 14a. Employing the immobilized magnetic particles 14Bc enables the analysis tool 1B to perform bioseparation quickly and easily. This enables automation of operations to be facilitated. In other respects, the analysis tool 1 B and the analysis device 2B of the present embodiment enable similar advantageous effects to be achieved to those of the second embodiment.

Fourth Embodiment

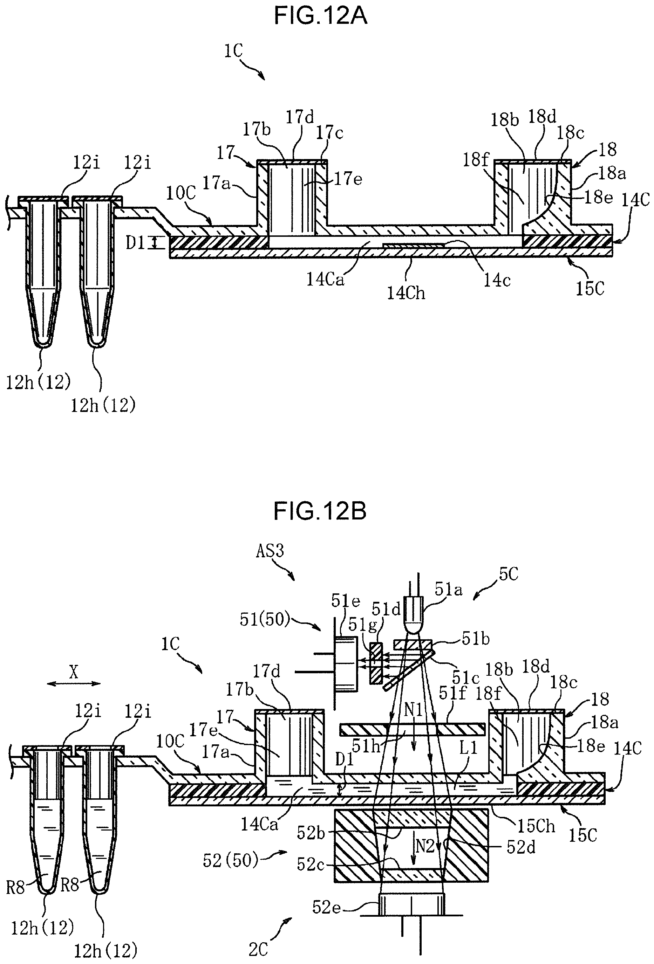

Next, explanation follows regarding an analysis tool 1C according to a fourth embodiment of technology disclosed herein, with reference to Fig. 12A and Fig. 12B. In the analysis tool 1C, configuration elements with functions the same as or similar to configuration elements of the analysis tool 1A of the second embodiment are allocated the same reference numerals. Detailed explanation of these configuration elements is omitted. The analysis tool 1C is employed with an analysis device 2C and an analysis system AS3. The analysis device 2C and the analysis system AS3 are substantially the same as the analysis device 2A and the analysis system AS 1 of the second embodiment, with the exception of the configurations described below, and so detailed explanation thereof is omitted.

As illustrated as an example in Fig. 12A, the analysis tool 1C differs from the analysis tool 1A in the point that the analysis tool 1C includes an upper base plate 10C, an adhesive layer 14C, an immobilization plate 15C, and a light measurement well 14Ca. Moreover, the analysis tool 1C differs from the analysis tool 1A in the point that the analysis tool 1C does not include a light measurement well on the immobilization plate 15C, and in the point that the analysis tool 1C does not include an opening through the upper base plate 10C and the adhesive layer 14C.

The upper base plate 10C forms an upper wall of the light measurement well 14Ca. As illustrated as an example in Fig. 12B, the light source section 51 illuminates the measurement solution L1 with excitation light in the illumination direction indicated by the arrow N1. The upper base plate 10C is accordingly formed using a material that allows the excitation light to pass through. Examples of the material of the upper base plate 10C include transparent or translucent resins such as polystyrene (PS), poly(methyl methacrylate) (PMMA), cyclo-olefin polymer (COP), polycarbonate (PC), low density polyethylene (LDPE), polylactic acid (PLA), polydimethylsiloxane (PDMS), and polypropylene (PP). Note that the upper base plate 10C corresponds to an example of a first base plate of technology disclosed herein.

As illustrated as an example in Fig. 12A, in the immobilization plate 15C, an antibody for the specified component in the biological sample S is immobilized as the immobilized antibody 14c. Examples of the material of the immobilization plate 15C include similar materials to those of the immobilization plate 15 of the analysis tool 1A. Immobilization of the antibody for the specified component is performed in a similar manner to with the immobilization plate 15 of the analysis tool 1A. The immobilization plate 15C corresponds to an example of a second base plate of technology disclosed herein.

The adhesive layer 14C is a member used to adhere the upper base plate 10C and the immobilization plate 15C together. The adhesive layer 14 is, for example, formed using double-sided tape. The adhesive layer 14C is colored black.

The light measurement well 14Ca is formed by punching adhesive layer 14C to form a punched out portion between the upper base plate 10C and the immobilization plate 15C. The light measurement well 14Ca links the inside 17e of the tube shaped protrusion 17 and the inside 18f of the tube shaped protrusion 18 together. In the analysis tool 1C, antigen-antibody reactions and enzyme reactions take place inside the light measurement well 14Ca. The size of the light measurement well 14Ca is set, for example, to approximate to a cuboid of length 30 mm $\times$ width 5 mm $\times$ height (depth) 0.15 mm. In such a configuration, the flow path capacity of the light measurement well 14Ca is 22.5 $\mu$L. The immobilization surface area of immobilized antibody 14c is, for example, 150 mm$^2$. Considering the reactivity between the biological sample S and the immobilized antibody 14c, the reactivity of the marker enzyme, and the fluorescent light emission strength, an appropriate amount of solution to be moved back and forth in the light measurement well 14Ca is, for example, 200 $\mu$L.

As illustrated as an example in Fig. 12B, the light measurement well 14Ca is formed flattened in the light receiving direction N2 of the light receiving element 52e. In the light measurement well 14Ca, the thickness of the adhesive layer 14C is the cell length D1. The cell length D1 is, for example, set to 3.0 mm or less, 1.5 mm to 3.0 mm, 1.9 mm to 2.5 mm, 2.5 mm, 2.0 mm, 1.5 mm to 2.0 mm, 1.9 mm to 2.0 mm, 2.0 mm to 2.5 mm, 2.0 mm to 3.0 mm, or 2.0 mm or less.

In the analysis tool 1C, the liquid L or measurement solution L1 in the light measurement well 14Ca is agitated by back and forth movement using the drawing/discharging nozzle 19, similarly to in the reaction flow path 14a of the analysis tool 1A. The second opening 18b is a location where the drawing/discharging nozzle 19 is inserted. The liquid L or the measurement solution L1 is moved back and forth inside the light measurement well 14Ca by drawing and discharging air. Note that movement back and forth of the liquid L or the measurement solution L1 may be performed by a combination of drawing and discharging the liquid L or the measurement solution L1 with the pipette tip 71, and drawing and discharging air with the drawing/discharging nozzle 19. The second opening 18b corresponds to an example of a solution transfer opening of technology disclosed herein.

As illustrated as an example in Fig. 12B, measurement of the measurement solution L1 in the light measurement well 14Ca is performed by a detection section 5C of the analysis device 2C. Unlike in the analysis device 2A, the detection section 5C includes an aperture 51f. The measurement solution L1 in the light measurement well 14Ca is illuminated with excitation light emitted from the light emitting element 51 a along the illumination direction N1. Fluorescent light emitted from the fluorophore in the measurement solution L1 excited by the excitation light is emitted from the measurement solution L1 along the light receiving direction N2 and received by the light receiving element 52e. The fluorescent light corresponds to an example of measurement light of technology disclosed herein. The measurement light may also be chemiluminescent light. In such cases, there is no need for excitation light illumination. Note that the measurement light is emitted through a lower face 15Ch of the immobilization plate 15C. The lower face 15Ch corresponds to an example of an emission section of technology disclosed herein.

The analysis tool 1C is configured so as to be capable of moving a solution back and forth and measuring the measurement solution L1 in the light measurement well 14Ca. Accordingly, the analysis tool 1C enables simplification of the structure. This thereby enables a reduction in size and reduction in manufacturing costs of the analysis tool 1C. In other respects, the analysis tool 1C and the analysis device 2C are capable of achieving similar advantageous effects to those of the second embodiment.

Fifth Embodiment

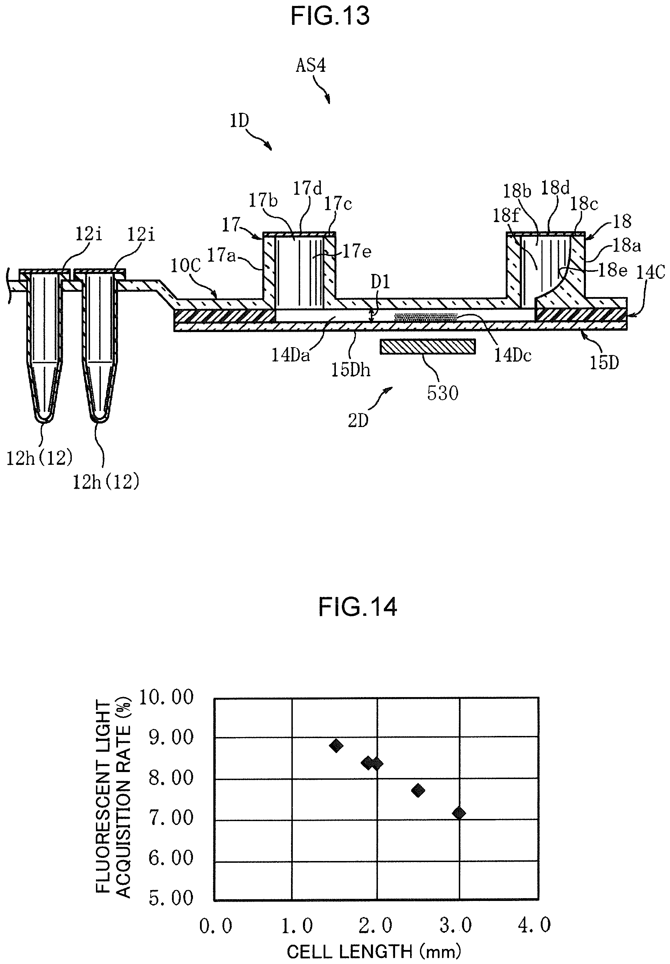

Next, explanation follows regarding an analysis tool 1D according to a fifth embodiment of technology disclosed herein, with reference to Fig. 13. In the analysis tool 1D, configuration elements with functions the same as or similar to those of configuration elements of the analysis tool 1C of the fourth embodiment are allocated the same reference numerals. Detailed explanation of these configuration elements is omitted. The analysis tool 1D is employed with an analysis device 2D and an analysis system AS4. The analysis device 2D and the analysis system AS4 are substantially the same as the analysis device 2C and the analysis system AS3 of the fourth embodiment, with the exception of the configurations described below, and so detailed explanation thereof is omitted.

The analysis tool 1D differs from the analysis tool 1C in the point that the analysis tool 1D includes a plate 15D, a light measurement well 14Da, and immobilized magnetic particles 14Dc. The plate 15D corresponds to an example of a first base plate of technology disclosed herein. In the analysis tool 1D, the antibody is not directly immobilized on the plate 15D. The immobilized magnetic particles 14Dc are disposed on the plate 15D inside the light measurement well 14Da. Note that a solution of antibody-sensitized magnetic particles may be prepared as a separate reagent rather than disposing the immobilized magnetic particles 14Dc on the plate 15D in the light measurement well 14Da from the outset.

In the analysis device 2D, the magnet 530 is placed below the analysis tool 1D. The magnet 530 keeps the immobilized magnetic particles 14Dc within the light measurement well 14Da by magnetic force, as the liquid L and the measurement solution L1 are transferred, and moved back and forth. Specific examples of the magnet 530 include an electromagnet or a permanent magnet.

Transfer and back and forth movement of the liquid L and the measurement solution L1 inside the light measurement well 14Da of the analysis tool 1D are performed by the analysis device 2D, similarly to in the analysis tool 1C. Measurement of fluorescent light emitted from the measurement solution L1 of the analysis tool 1D is performed by the analysis device 2D, similarly to in the analysis tool 1C. The fluorescent light corresponds to an example of measurement light of technology disclosed herein. The measurement light may also be chemiluminescent light. In such cases, there is no need to illuminate the excitation light. Note that the measurement light is emitted through a lower face 15Dh of the immobilization plate 15D. The lower face 15Dh corresponds to an example of an emission section of technology disclosed herein.

The analysis tool 1D includes the immobilized magnetic particles 14Dc inside the light measurement well 14Da. Employing the immobilized magnetic particles 14Dc enables the analysis tool 1D to perform bioseparation quickly and easily. This facilitates automation of operations. In other respects, the analysis tool 1D and the analysis device 2D of the present embodiment enable similar advantageous effects to be achieved to those of the fourth embodiment.

Technology disclosed herein is not limited to the content of the embodiments described above. Specific configurations of analysis tools and analysis devices according to technology disclosed herein may be subject to various design modifications.

As described above, the technology disclosed herein may be applied not only to measuring fluorescent light, but also to measuring chemiluminescent light. In such cases, there is no need to illuminate the measurement solution L1 with excitation light in the analysis device 2, 2A, 2B, 2C, and 2D.

In the first to the fifth embodiments, the light measurement method employed by the analysis devices 2, 2A, 2B, 2C, 2D is TOP-BOTTOM light measurement. However, in the technology disclosed herein, the light measurement method may also employ BOTTOM-TOP light measurement. Such a configuration has the technical advantage that measurement light such as fluorescent light or chemiluminescent light is not attenuated by the synthetic resin material of the light measurement well.

In the first to the fifth embodiments, explanation has been given regarding examples in which, in cases in which the measurement light is fluorescent light, the illumination direction of the excitation light is aligned with the light receiving direction of the fluorescent light. The illumination direction of the excitation light is not limited thereto, and the measurement solution L1 may be illuminated with excitation light in any direction except for the direction of the emission section of the light measurement well 13, 13A, 14Ca, 14Da. Specifically, for example, configuration may be made in which the measurement solution L1 is illuminated with excitation light from a direction intersecting the light receiving direction. More specifically, configuration may be made in which the measurement solution L1 is illuminated with excitation light from a direction orthogonal to the light receiving direction.

In the first to the fifth embodiments, explanation has been given regarding cases in which the antibody is respectively immobilized at the inner face 11e of the reaction tube 11, the immobilization plate 15, 15C, and the immobilized magnetic particles 14Bc, 14Dc. However, for example, in cases in which the analysis tool 1, 1A, 1B, 1C, 1D is employed to analyze an antibody as the specified component in the biological sample S, configuration may be made in which antigens for the antibody are immobilized on the plate or the magnetic particles.

In the second to the fifth embodiments, the analysis system AS 1, AS2, AS3, AS4 may be configured to use an immobilization plate or immobilized magnetic particles according to circumstances, and to be capable measuring with both. For example, in the case of an analysis system provided with plural measurement channels, it is possible to suppress an increase in the cost of the analysis device if only some specified measurement channels are made compatible with immobilized magnetic particles.

In the technology disclosed herein, in cases in which the light measurement well has a shape of, for example, width 2.0 mm x length 9.0 mm $\times$ height (depth) 9.0 mm, a detection method is also possible with measurement light at a 90° angle with respect to the excitation light. In such cases too, the measurement light is still emitted with a flattened face as an emission section.

In the first to the fifth embodiments, the analysis system AS, AS 1, AS2, AS3, AS4 includes the light guide 52a. However, technology disclosed herein may be configured with the light guide 52a omitted. Configuring in this manner enables a reduction in the number of configuration components, thereby enabling a reduction in the manufacturing cost of the analysis device.

Examples

Specific explanation follows regarding advantageous effects of the first to the fifth embodiments, based on Examples. Note that the technology disclosed herein is not limited by these Examples.

Example 1

Table 1 and Fig. 14 illustrate measurement results for fluorescent light harvesting rate for cases in which the light measurement well 13 of the first embodiment is set with a diameter D2 of 11.3 mm, 10.0 mm, 9.8 mm, 8.8 mm, and 8.0 mm, and the corresponding cell length D1 is set to 1.5 mm, 1.9 mm, 2.0 mm, 2.5 mm, and 3.0 mm, respectively. A solution containing 0.6 mM 4-MU (material obtained by marker enzyme AP cleavage of the fluorogenic substrate 4-MUP) was employed as the fluorophore. The dispensing amount was 150 $\mu$L. The central wavelength of the excitation light was 365 nm, and the detection wavelength was 450 nm.

As is apparent from Table 1 and Fig. 14, the fluorescent light harvesting rate increases the larger the diameter D2, and the shorter the cell length D1. It is therefore apparent that the shape of the measurement solution L1 dispensed into the light measurement well 13 is preferably flattened in the light receiving direction, and a cell length D1 of 3.0 mm or less is appropriate. In particular, the shape of the portion that the measurement solution L1 enters is preferably Φ9.8 $\times$ 2 mm. This also applies for the analysis tools 1A, 1B, 1C, 1D of the second to fifth embodiments.

Table 1

| Diameter (mm) | Cell length (mm) | Fluorescent light harvesting rate |
|---|---|---|
| 11.3 | 1.5 | 8.87% |
| 10.0 | 1.9 | 8.48% |
| 9.8 | 2.0 | 8.45% |
| 8.8 | 2.5 | 7.76% |
| 8.0 | 3.0 | 7.22% |

Example 2

In the first embodiment, confirmation was carried out as to whether or not the prozone effect arises when excess fluorogenic substrate is present in cases in which the shape of the measurement solution L1 dispensed into the light measurement well 13 is flattened in the light receiving direction N2. Diluted solutions of 4-MU were employed as the measurement solution L1. The concentrations of the diluted solutions of 4-MU were 0.4, 4, 40, 400, and 4000 μM. The dispensed amount was 150 μL. Measurement was performed with the central wavelength of the excitation light at 365 nm, and the detection wavelength at 450 nm. The shape of the diluted solution of 4-MU dispensed in the light measurement well 13 was a flat, circular plate shape of diameter (D2) of 9.8 mm × cell length (D1) of 2.0 mm. Note that condition 1 refers to feedback resistance of AD output for the light receiving section being set to 47 MΩ. Condition 2 refers to feedback resistance of AD output for the light receiving section being set to 4.7 MΩ.

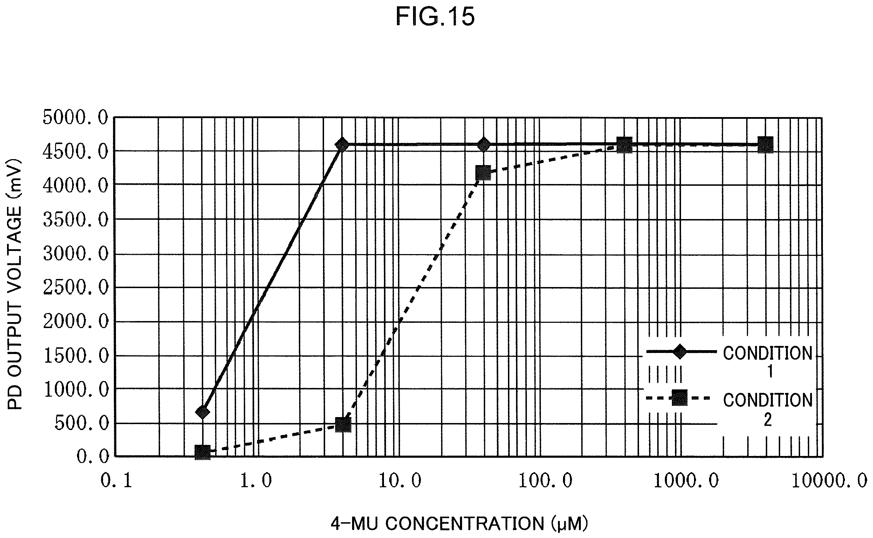

As illustrated in Fig. 15, the output voltage value remained saturated, at approximately 4600 mV, up to a 4-MU concentration of 4000 μM under both condition 1 and condition 2. It is accordingly apparent that in the light measurement well 13, a values turning lower due to the prozone effect (false negatives) do not arise within a realistic concentration range of the 4-MU. This is since absorption loss of the excitation light does not readily occur since the cell length D1 is sufficiently short as a result of the flattened profile of the light measurement well 13. This is also true of the analysis tools 1A, 1B, 1C, and 1D of the second to the fifth embodiments.

Example 3

In the first embodiment, the relationship between the cell length D1 and a dynamic range high limit was confirmed in cases in which the shape of the measurement solution L1 dispensed into the light measurement well 13 is flattened in the light receiving direction. A diluted solution of 4-MU was employed as the measurement solution L1. The internal diameter of the light measurement well 13 was 9.8 mm, and diluted solutions of 4-MU were dispensed in three amounts: 150 μL, 100 μL, and 75 μL. In each of these cases, the cell length D1 after dispensing into the light measurement well 13 was 2.0 mm, 1.33 mm, and 1.0 mm, respectively. The diluted solutions of 4-MU were configured with concentrations of 0, 4, 40, 400, 4000, 40000 nM for each amount dispensed. A dynamic range high limit (HL) for the cell length D1 is found using the following Equation 1, employing a slope (a') of a linear approximation equation derived from average values of the AD output voltage values (mV) for each concentration, a y-intercept (b'), and a maximum output value of 4500 mV for the AD.

Equation 1

$$HL = \frac{4500 - b'}{a'}$$

Fig. 16 illustrates a relationship between cell length and dynamic range high limit. When the cell length D1 is changed from 2.0 mm to 1.0 mm, the dynamic range increases by a factor of approximately 1.8. It is accordingly apparent that the dynamic range high limit can be raised by shortening the cell length. As is apparent from the results of Example 3, in the light measurement well 13, values turning lower due to the prozone effect (false negatives) do not arise. Accordingly, saturation of the AD output values is guaranteed to be due to high concentration values in excess of the dynamic range high limit. For a cell length D1 of 2.0 mm during normal measurement, quantification is clearly possible by the device automatically determining when the output value of the AD has become saturated, using the nozzle 7 to draw some of the measurement solution L1 from the light measurement well 13 so as to change to a predetermined cell length D1, and then re-measuring. This is also true of the analysis tools 1A and 1B of the second and third embodiments.

All cited documents, patent applications, and technical standards mentioned in the present specification are incorporated by reference in the present specification to the same extent as if the individual cited document, patent application, or technical standard was specifically and individually indicated to be incorporated by reference.

**Claims**

1. An analysis tool configured to be mounted to an analysis device that automatically analyzes a specified component contained in a sample, the analysis tool comprising:

   a light measurement well that holds a measurement solution as a measurement subject, and that measures the measurement solution, the light measurement well including:

   an opening that dispenses the measurement solution,
   a measurement solution holder that holds the measurement solution dispensed via the opening, and
   an emission section that emits measurement light in a light receiving direction of the analysis device, the measurement light caused to be emitted from the measurement solution held in the measurement solution holder,
   wherein the measurement light is fluorescent light or chemiluminescent light, and
   wherein the measurement solution holder has a flattened profile that is flattened in the light receiving direction.

2. The analysis tool of claim 1, wherein:

   the light measurement well includes a flattened face and is formed flattened overall so as to match a shape of the measurement solution holder; and
   the emission section is provided at the flattened face.

3. The analysis tool of claim 1 or 2, wherein:

   a height of the measurement solution in a crosswise direction of a cross-section of the flattened profile defines a cell length of the light measurement well; and
   the cell length is 3 mm or less.

4. The analysis tool of any one of claims 1 to 3, further comprising a reaction tube that generates the measurement solution.

5. The analysis tool of any one of claims 1 to 4, wherein, in cases in which the measurement light is fluorescent light, excitation light to generate the measurement light is illuminated onto the measurement solution from a portion other than the emission section of the light measurement well.

6. The analysis tool of claim 5, wherein an illumination direction of the excitation light is aligned with the light receiving direction of the measurement light.

7. An analysis device comprising the analysis tool of any one of claims 1 to 6, the analysis device comprising a detector that detects the measurement light.

8. The analysis device of claim 7, further comprising a measurement light wavelength selection filter that limits wavelengths of the measurement light.

9. The analysis device of claim 8, wherein the measurement light wavelength selection filter has predetermined wavelength absorption characteristics.

10. The analysis device of claim 9, wherein the measurement light wavelength selection filter is a colored glass filter.

11. The analysis device of any one of claims 7 to 10, further comprising a light collecting member to collect the measurement light.

**12.** The analysis device of claim 11, wherein the light collecting member is a light guide.

**13.** The analysis device of any one of claims 7 to 12 that, in cases in which the measurement light is fluorescent light, further comprises:

a light source that illuminates excitation light to cause the measurement solution to emit fluorescent light; and an excitation light wavelength selection filter that limits wavelengths of the excitation light.

**14.** The analysis device of claim 13, wherein the excitation light wavelength selection filter has predetermined wavelength absorption characteristics.

**15.** The analysis device of claim 13 or 14, wherein the excitation light wavelength selection filter is a colored glass filter.

**16.** The analysis device of any one of claims 7 to 15, wherein:

the analysis tool is configured such that a cell length of the light measurement well is determined by a height of the measurement solution in a crosswise direction of a cross-section of the flattened profile, and the cell length of the light measurement well can be changed by changing an amount of the measurement solution, and the analysis device further comprises:

a nozzle; and
a controller that controls operation of the nozzle and the detector, the controller being configured so as to, in cases in which an output value of the detector has become saturated, reduce the amount of the measurement solution by drawing the measurement solution into the nozzle to shorten the cell length, and to then cause the detector to re-execute measurement.

**17.** The analysis device of claim 16, wherein an amount by which to reduce the amount of the measurement solution is determined based on time taken from detecting the measurement light with the detector until the output value of the detector reaches a saturated state.

**18.** The analysis device of claim 17, wherein the amount by which to reduce the amount of the measurement solution is determined based on time taken from detecting the measurement light with the detector until the output value of the detector reaches a saturated state, and a rate of rise of the output value of the detector during a predetermined period that has been predetermined as an initial reaction period of the measurement solution.

# FIG.1

# FIG.2A

# FIG.2B

FIG.3A

CROSSWISE
DIRECTION

# FIG.3B

LENGTHWISE
DIRECTION

# FIG.4A

13b
13c
13d
13
IVB
13a
IVB

# FIG.4B

13b
13f
N1
13c
13a
13d
D1
13g
13h
N2
13e
D2

CROSS-SECTION
ALONG IVB-IVB

# FIG.5A

# FIG.5B

FIG.6

# FIG.7

FIG.8

# FIG.9A

# FIG.9B

CROSS-SECTION
ALONG IXB-IXB

# FIG.10A

EP 3 206 013 A1

FIG.10B

# FIG.11

# FIG.12A

# FIG.12B

## FIG.13

## FIG.14

# FIG.15

EP 3 206 013 A1

## FIG.16

Graph: X-axis: FLUOROGENIC SUBSTRATE AMOUNT (MEASUREMENT SOLUTION AMOUNT) (μL), values 70, 90, 110, 130, 150. Y-axis: DYNAMIC RANGE UPPER LIMIT (μM), values 0.0 to 90.0.

CELL LENGTH 1 mm

CELL LENGTH 1.33 mm

CELL LENGTH 2 mm

$$y = 2832.2x^{-0.828}$$

## FIG.17

ANALYSIS DEVICE — 2, 40

CONTROLLER

CPU 100

RAM 102

ROM 104 / 106(108)

MEASUREMENT PROGRAM

CONTROL LINES

DETECTION SECTION 5

DISPENSING SECTION 6

INPUT SECTION 42

DISPLAY SECTION 43

## FIG.18

OUTPUT
VALUE

CONCENTRATION

O

SATURATED VALUE

## FIG.19

OUTPUT
VALUE

CONCENTRATION

O

FIRST
CONCENTRATION  SECOND
CONCENTRATION  SATURATED
VALUE

# FIG.20

Flowchart:

MEASUREMENT PROCESSING

START MEASUREMEN — 200

202 — IS OUTPUT VALUE > 0?
N → (loop back)
Y ↓

204 — NON-SATURATED STATE?
N → END MEASUREMENT — 208
Y ↓

206 — HAS END CONDITION BEEN SATISFIED?
N → (loop back)
Y ↓ END

208 — END MEASUREMENT
210 — DERIVE MEASUREMENT SOLUTION REDUCTION AMOUNT
212 — REDUCE MEASUREMENT SOLUTION

# FIG.21

```
           ( MEASUREMENT PROCESSING )
                       │
                       │◄──────────────────────────┐
                ┌──────▼──────┐                     │
                │    START    │                     │
                │  MEASUREMEN │ ~200                │
                └──────┬──────┘                     │
                       │                            │
          ┌───────────►│                            │
          │            ▼           202              │
          │        ╱─────────╲                      │
        N │      ╱  IS OUTPUT  ╲                     │
          │◄────◄    VALUE > 0?  ►                   │
          │      ╲             ╱                     │
          │        ╲─────────╱                       │
          │            │ Y                           │
          │            ▼        204                  │
          │        ╱─────────╲                       │
          │      ╱ NON-SATURATED╲    N         ┌─────▼──────┐
          │     ◄    STATE?      ►──────────►  │    208     │
          │      ╲             ╱               │    END     │
          │        ╲─────────╱                 │MEASUREMENT │
          │            │ Y        206          └─────┬──────┘
          │            ▼                             │   300
          │        ╱─────────╲                       ▼
        N │      ╱  HAS END    ╲              ┌──────────────┐
          │◄────◄ CONDITION BEEN►             │REDUCE MEASURE│
                 ╲  SATISFIED? ╱              │MENT SOLUTION │
                   ╲─────────╱                │ BY SPECIFIC  │
                       │ Y                    │   AMOUNT     │
                   ┌───▼───┐                  └──────────────┘
                   │  END  │
                   └───────┘
```

# FIG.22

```
        2                                    400
        │                                     │
   ┌────▼────┐                    ┌───────────▼──────────┐
   │         │                    │   STORAGE MEDIUM     │  106(108)
   │ ANALYSIS│   INSTALL          │  ┌─────────────────┐ │
   │ DEVICE  │◄ - - - - - - - - - │  │  MEASUREMENT    │ │
   │         │                    │  │   PROGRAM       │ │
   │         │                    │  └─────────────────┘ │
   └─────────┘                    └──────────────────────┘
```

Europäisches
Patentamt
European
Patent Office
Office européen
des brevets

# EUROPEAN SEARCH REPORT

Application Number

EP 17 15 3495

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2 663 801 A (MORRIS SLAVIN ET AL) 22 December 1953 (1953-12-22) * column 1, line 1 - line 5 * * column 2, line 44 - line 50 * * column 3, line 36 - line 69 * * column 4, line 3 - line 71 * | 1-10, 13-15 | INV. G01N21/11 G01N21/64 G01N21/76 G01N35/10 |
| X | JP 2008 096407 A (OLYMPUS CORP) 24 April 2008 (2008-04-24) * abstract * * paragraphs [0021], [0024], [0031], [0040] * * figures 13,17 * | 1,2,5-9, 11,13,14 | |
| X | US 4 922 092 A (RUSHBROOKE JOHN [GB] ET AL) 1 May 1990 (1990-05-01) * column 3, line 11 - line 13 * * column 8, line 41 - column 9, line 19 * * column 9, line 38 - line 45 * * figure 3 * | 1-5,7-9, 11-15 | |
| Y | | 16-18 | |
| Y | US 6 468 803 B1 (BERNDT KLAUS W [US]) 22 October 2002 (2002-10-22) * column 7, line 7 - line 46 * * figures 1,3,4 * | 16-18 | TECHNICAL FIELDS SEARCHED (IPC) G01N |
| Y | US 5 478 526 A (SAKAI TADASHI [JP] ET AL) 26 December 1995 (1995-12-26) * column 9, line 28 - line 43 * * figures 3,5 * | 16-18 | |
| A | WO 2012/022482 A1 (ETH ZUERICH [CH]; VOEROES JANOS [CH]; ZENOBI-WONG MARCY [CH]; PRAYANKA) 23 February 2012 (2012-02-23) * page 17, line 21 - page 18, line 8 * | 1-18 | |

The present search report has been drawn up for all claims

| Place of search | Date of completion of the search | Examiner |
|---|---|---|
| Munich | 23 June 2017 | D'Alessandro, Davide |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 15 3495

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2017

| Patent document cited in search report | | Publication date | Patent family member(s) | | Publication date |
|---|---|---|---|---|---|
| US 2663801 | A | 22-12-1953 | NONE | | |
| JP 2008096407 | A | 24-04-2008 | JP | 4889437 B2 | 07-03-2012 |
| | | | JP | 2008096407 A | 24-04-2008 |
| US 4922092 | A | 01-05-1990 | NONE | | |
| US 6468803 | B1 | 22-10-2002 | EP | 1260810 A2 | 27-11-2002 |
| | | | JP | 2003130795 A | 08-05-2003 |
| | | | MX | PA02005109 A | 11-08-2004 |
| | | | US | 6468803 B1 | 22-10-2002 |
| US 5478526 | A | 26-12-1995 | DE | 4310607 A1 | 14-10-1993 |
| | | | JP | 3152727 B2 | 03-04-2001 |
| | | | JP | H05281243 A | 29-10-1993 |
| | | | US | 5478526 A | 26-12-1995 |
| WO 2012022482 | A1 | 23-02-2012 | EP | 2606336 A1 | 26-06-2013 |
| | | | US | 2013244895 A1 | 19-09-2013 |
| | | | WO | 2012022482 A1 | 23-02-2012 |

EPO FORM P0459

For more details about this annex : see Official Journal of the European Patent Office, No. 12/82

**REFERENCES CITED IN THE DESCRIPTION**

**Patent documents cited in the description**

- JP H10019779 A

- JP 2000241708 A